# EUROPEAN PATENT SPECIFICATION

(11) **EP 3 602 068 B1**
(45) Date of publication and mention of the grant of the patent: **18.10.2023**
(21) Application number: 18715247.5
(22) Date of filing: 28.03.2018
(51) Int. Cl.: G01N 33/68

(54) **BIOMARKER FOR PREDICTING COGNITIVE DECLINE**
BIOMARKER ZU VORHERSAGE KOGNITIVEN VERFALLS
BIOMARQUEUR POUR PRÉDIRE DU DÉCLIN COGNITIF

(30) Priority: 29.03.2017 GB 201705044; 06.07.2017 GB 201710858
(43) Date of publication of application: 05.02.2020
(73) Proprietor: Neuro-Bio Ltd, Abingdon, Oxfordshire OX14 3DB (GB)
(72) Inventor: GREENFIELD, Susan, Abingdon Oxfordshire OX14 3DB (GB); TU, Henry, Abingdon Oxfordshire OX14 3DB (GB); MORRILL, Paul, Abingdon Oxfordshire OX14 3DB (GB); ROTONDO, Sergio, Abingdon Oxfordshire OX14 3DB (GB); GARCIA-RATES, Sara, Abingdon Oxfordshire OX14 3DB (GB)
(74) Representative: Hutter, Anton
(86) International application number: PCT/GB2018/050817
(87) International publication number: WO 2018/178665

(56) References cited:
- WO-A1-2016/156803
- WO-A2-2017/130003
- MORRILL PAUL ET AL: "A NOVEL PEPTIDE PIVOTAL IN ALZHEIMER'S DISEASE: A POTENTIAL BIOMARKER IN HUMAN BLOOD POSSIBLY LINKED TO COGNITIVE DECLINE?", ALZHEIMER'S & DEMENTIA: THE JOURNAL OF THE ALZHEIMER'SASSOCIATION, vol. 13, no. 7, 19 July 2017 (2017-07-19), XP085217687, ISSN: 1552-5260, DOI: 10.1016/J.JALZ.2017.06.1929
- HALLIDAY A C ET AL: "Evaluation of a technique to identify acetylcholinesterase C-terminal peptides in human serum samples", CHEMICO-BIOLOGICAL INTERACTIONS, ELSEVIER SCIENCE IRLAND, IR, vol. 187, no. 1-3, 6 September 2010 (2010-09-06), pages 110-114, XP027174326, ISSN: 0009-2797 [retrieved on 2010-02-13]

## Description

The disclosure relates to biomarkers, and particularly, although not exclusively, to biomarkers for neurodegenerative disorders, such as Alzheimer's disease, Parkinson's disease, Huntington's disease or Motor Neurone disease. The disclosure especially relates to a novel quantitative predictive biomarker for cognitive decline, and in particular the rate of cognitive decline that a subject will suffer, for example as a result of developing a neurodegenerative disorder. The disclosure further provides diagnostic and prognostic methods, and kits for predicting the rate of cognitive decline in subjects.

Alzheimer's disease (AD) is fast becoming one of the biggest socioeconomic burdens in the world with a growing worldwide incidence rate of almost 10 million new cases of dementia each year. Since both the incidence and prevalence of AD increases with age, the number of patients is growing significantly with an aging population. In 2015, there were over 46 million people living with dementia with an estimated socioeconomic cost of $800 billion annually and it is expected that the number of patients will rise to over 130 million by 2050 costing society over $2 trillion annually. AD was recently announced as the UK's leading cause of death in the over 65's, and in the US it is now the 6th leading cause of death across all ages.

At present there exists no single test that can be administered to diagnose Alzheimer's disease. Clinical classification of Alzheimer's disease relies on a combination of subjective reporting, medical history evaluation, cognitive function tests, and costly brain imaging scans with no true classification possible until a post-mortem examination of the brain can be conducted. The identification of a biomarker that allows objective detection of AD or rate of cognitive decline with a high degree of specificity and sensitivity would allow routine screening, early intervention, and revolutionise neuropathological research by opening the pathway for new strategies in intervention.

The inventors have continued their previous work in this area, and have focused on the toxic peptide, T14, which is derived from the C-terminus of acetylcholinesterase (AChE), which is present as a naturally occurring bioactive molecule in brain tissue. WO 2016/156803 describes an antibody raised against T14 which can selectively recognise and quantify this innate signalling molecule in either human and rat brain tissue or blood samples, by ELISA or Western blotting techniques. Now, employing the same procedure and methods, the inventors have analysed a statistically significant number of human plasma samples, which have surprising shown that varying AChE-derived peptide (i.e. T14) levels correlates with age, and can be used as an objective and quantifiable predictor or biomarker of cognitive decline.

The invention is as defined by the claims and any other aspects, configurations or embodiments set forth herein not falling within the scope of the claims are for information only. Additionally, any references in the description to methods of treatment refer to the compounds, pharmaceutical compositions, and medicaments of the present disclosure for use in a method of treatment of the human (or animal) body by therapy (or for diagnosis).

Hence, in a first example, there is disclosed a method for predicting the rate at which a subject will suffer from cognitive decline, or for detecting a pre-disposition thereto, the method comprising:-
(a) analysing, in a sample obtained from a test subject, the concentration of (i) a soluble peptide comprising or consisting of SEQ ID No:3, or a variant or fragment thereof and/or (ii) an aggregated peptide comprising or consisting of SEQ ID No:3, or a variant or fragment thereof; and
(b) comparing this concentration with a reference value from a control population for concentrations of either soluble or aggregated peptide comprising or consisting of SEQ ID No:3, or a variant or fragment thereof,
wherein a lower concentration of soluble peptide comprising or consisting of SEQ ID No:3, or a variant or fragment thereof, and/or a higher concentration of aggregated peptide comprising or consisting of SEQ ID No:3, from the test subject, relative to the respective reference value, suggests that the subject will suffer from a higher rate of cognitive decline, or has a pre-disposition thereto.

In a second example, there is disclosed a cognitive decline prediction kit, for identifying a subject prone to suffering from cognitive decline, or having a pre-disposition thereto, the kit comprising:
(a) means for determining, in a sample obtained from a test subject, the concentration of (i) a soluble peptide comprising or consisting of SEQ ID No:3, or a variant or fragment thereof and/or (ii) an aggregated peptide comprising or consisting of SEQ ID No:3, or a variant or fragment thereof; and
(b) a reference value from a control population for concentrations of either soluble or aggregated peptide comprising or consisting of SEQ ID No:3, or a variant or fragment thereof,
wherein the kit is used to identify a lower concentration of soluble peptide comprising or consisting of SEQ ID No:3, or a variant or fragment thereof, and/or a higher concentration of aggregated peptide comprising or consisting of SEQ ID No:3, in the sample from the test subject, compared to the respective reference value, thereby suggesting that the test subject will suffer from a higher rate of cognitive decline, or has a pre-disposition thereto.

In a third example, there is disclosed a method of treating a subject prone to suffering from cognitive decline, or having a pre-disposition thereto, the method comprising:-
(a) analysing, in a sample obtained from the subject, the concentration of (i) a soluble peptide comprising or consisting of SEQ ID No:3, or a variant or fragment thereof and/or (ii) an aggregated peptide comprising or consisting of SEQ ID No:3, or a variant or fragment thereof, wherein a lower concentration of soluble peptide comprising or consisting of SEQ ID No:3, or a variant or fragment thereof, and/or a higher concentration of aggregated peptide comprising or consisting of SEQ ID No:3, from the test subject, relative to a respective reference value from a control population for concentrations of either soluble or aggregated peptide comprising or consisting of SEQ ID No:3, or a variant or fragment thereof, suggests that the subject will suffer from a higher rate of cognitive decline, or has a pre-disposition thereto; and
(b) administering, to the subject, a therapeutic agent that prevents, reduces or delays cognitive decline.

Advantageously, the research described herein supports the notion that detection of the peptide of SEQ ID No:3 (referred to herein as "T14") in individuals can be used in the first ever objective diagnostic or prognostic test for predicting whether or not a subject will suffer from a higher rate of cognitive decline, as a result of developing a neurodegenerative disorder, compared to an otherwise healthy individual. Use of the T14 biomarker (i.e. the peptide of SEQ ID No:3, or a variant or fragment thereof) allows detection of the susceptibility to a higher rate of cognitive decline with a very high degree of specificity and sensitivity from a non-invasive, easily repeated, cost-effective procedure, such as blood collection, and therefore allows routine screening, and early intervention with therapeutic treatment.

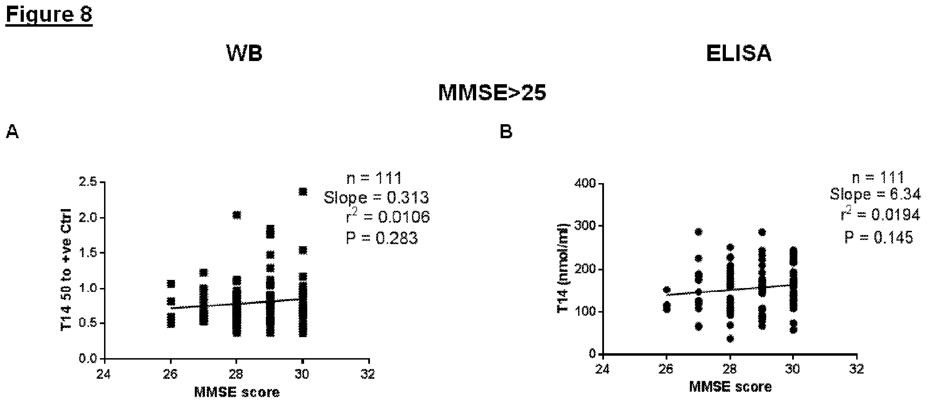

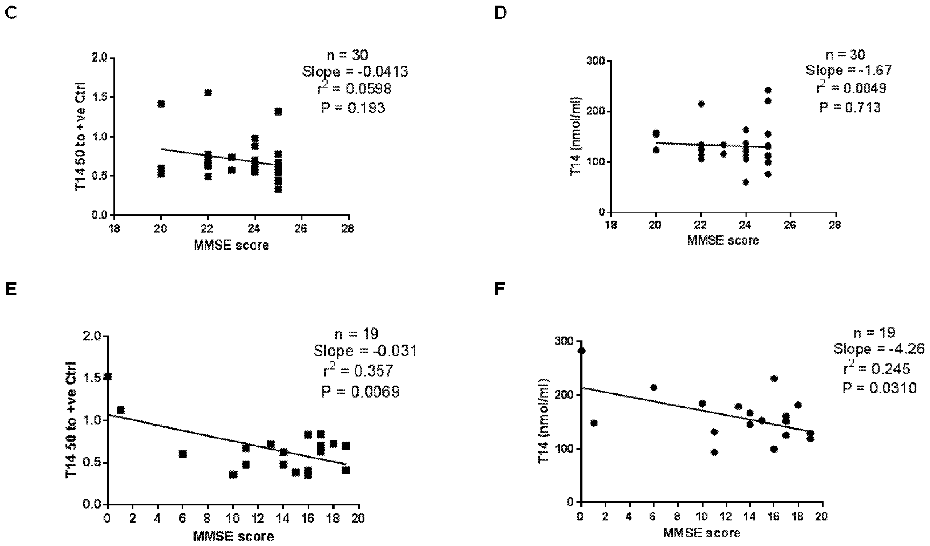

As described in Example 4 and in Figure 8, the clear correlation between T14 levels (soluble and/or aggregated forms) and MMSE scores of less than 20 corroborate the T14 assay data, thereby confirming that T14 levels can be reliably used to predict whether or not a test subject will suffer from cognitive decline in later years, way before they ever show any symptoms, as is currently the case using standard MMSE scoring. This means that the methods described herein, which detect T14, can be used as an early warning test, such that positive steps can then be taken to reduce the risk of cognitive decline (e.g. diet, life style choices), and administration of therapies, as appropriate.

It will be appreciated that the method according to the first example is useful for enabling a clinician to make decisions with regards to the best course of treatment for a subject who is at risk of suffering from a higher rate of cognitive decline in later life. Preferably, the method is carried out before symptoms of cognitive decline have developed, for example when the subject is in their 30's, 40's, 50's, and 60's to detect if the subject might later suffer from a higher rate of cognitive decline, and then, if a negative prognosis is detected, begin treatment to prevent onset of the cognitive decline. As such, the condition is prevented before symptoms ever appear. In addition, the method of the first example is useful for monitoring the efficacy of a putative treatment for cognitive decline. Hence, the kit according to the second example is useful for providing a prognosis of the subject's condition, such that the clinician can carry out the treatment according to the third example. The kit may also be used to monitor the efficacy of a putative treatment for cognitive decline. The method and the kit are therefore very useful for guiding a cognitive decline treatment regime for the clinician, and to monitor the efficacy of such a treatment regime.

Examples of suitable therapeutic agents which may be administered to the subject to prevent or treat the cognitive decline include, but are not limited to, acetylcholinesterase inhibitors, such as Rivastigmine, Galantamine, and Donepezil, and/or N-methyl-D-aspartate (NMDA) antagonists, such as Memantine.

Therefore, the concentration of the T14 peptide, either in its soluble or aggregated form, may act as a diagnostic and/or prognostic marker for cognitive decline.

Thus, in a fourth example, there is disclosed use of a peptide comprising or consisting of SEQ ID No:3, or a variant or fragment thereof, as a diagnostic or prognostic biomarker for predicting the rate of cognitive decline.

Most preferably, the method of the first example is a prognostic method, and the peptide is therefore used in the fourth example as a prognostic biomarker. The disclosure may be used in a diagnostic or prognostic method to diagnose or predict the rate of cognitive decline caused by any neurodegenerative disease selected from a group consisting of: Alzheimer's disease; Parkinson's disease; Huntington's disease; Motor Neurone disease; Spinocerebellar type 1, type 2, and type 3; Amyotrophic Lateral Sclerosis (ALS); schizophrenia; Lewy-body dementia; and Frontotemporal Dementia. It is preferred, however, that the disclosure is used to study or predict cognitive decline in any neurological disorder associated with non-enzymatic function of AChE, in particular, for example, Alzheimer's Disease, Parkinson's Disease and Motor Neuron Disease.

However, it is especially preferred that the disclosure is used to predict the rate of cognitive decline in an Alzheimer's Disease patient.

Following on from their previous studies, the inventors continued their research with acetylcholinesterase, and their antibody which exhibits immunospecificity to specific regions in the C-terminus of this enzyme, as described in WO 2016/156803. Acetylcholinesterase is a serine protease that hydrolyses acetylcholine, and is well-known to the skilled person. The major form of acetylcholinesterase which is found in the brain is known as tailed acetylcholinesterase (T-AChE). The protein sequence of one embodiment of human tailed acetylcholinesterase (Gen Bank: AAA68151.1) is 614 amino acids in length, and is provided herein as SEQ ID No:1, as follows:

1 mrppqcllht pslaspllll llwllgggvg aegredaell vtvrggrlrg irlktpggpv
61 saflgipfae ppmgprrflp pepkqpwsgv vdattfqsvc yqyvdtlypg feqtemwnpn
121 relsedclyl nvwtpyprpt sptpvlvwiy gggfysgass ldvydgrflv qaertvlvsm
181 nyrvgafgfl alpgsreapg nvglldqrla lqwvqenvaa fggdptsvtl fgesagaasv
241 gmhllsppsr glfhravlqs gapngpwatv gmgearrrat qlahlvgcpp ggtggndtel
301 vaclrtrpaq vlvnhewhvl pqesvfrfsf vpvvdgdfls dtpealinag dfhglqvlvg
361 vvkdeqsyfl vygapgfskd neslisraef laqvrvgvpq vsdlaaeavv lhytdwlhpe
421 dparlreals dvvgdhnvvc pvaqlagrla aqgarvyayv fehrastlsw plwmgvphgy
481 eiefifgipl dpsrnytaee kifaqrlmry wanfartgdp neprdpkapq wppytagaqq
541 yvsldlrple vrrglraqac afwnrflpkl lsatdtldea erqwkaefhr wssymvhwkn
601 qfdhyskqdr csdl

[SEQ ID No:1]

The amino acid sequence of T30 (which corresponds to the last 30 amino acid residues of SEQ ID No:1) is provided herein as SEQ ID No:2, as follows:-
KAEFHRWSSYMVHWKNQFDHYSKQDRCSDL
[SEQ ID No:2]

The amino acid sequence of T14 (which corresponds to the 14 amino acid residues located towards the end of SEQ ID No:1, and lacks the final 15 amino acids found in T30) is provided herein as SEQ ID No:3, as follows:-
AEFHRWSSYMVHWK
[SEQ ID No:3]

Preferably, therefore, the peptide of SEQ ID No:3, or a variant or fragment thereof is T14.

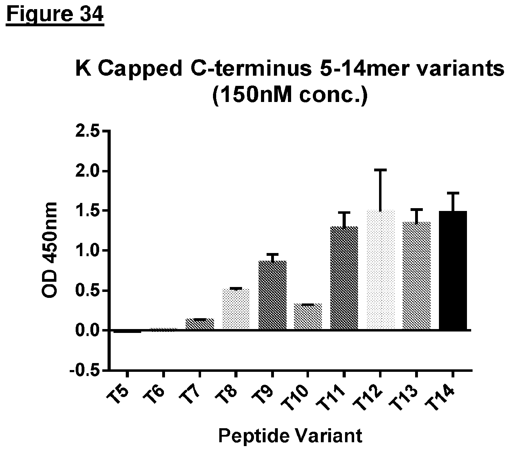

Fragments of T14 (SEQ ID No:3) are also detectable as shown in Example 7 and Figure 34, and can act as diagnostic or prognostic markers used in accordance with the disclosure.

Thus, in one example, a fragment of SEQ ID No:3 preferably comprises an amino acid sequence of SEQ ID No:7 (i.e. T7), i.e. SYMVHWK.

In another example, a fragment of SEQ ID No:3 preferably comprises an amino acid sequence of SEQ ID No: 8 (i.e. T8), i.e. SSYMVHWK.

In another example, a fragment of SEQ ID No:3 preferably comprises an amino acid sequence of SEQ ID No: 9 (i.e. T9), i.e. WSSYMVHWK.

In another example, a fragment of SEQ ID No:3 preferably comprises an amino acid sequence of SEQ ID No: 10 (i.e. T10), i.e. RWSSYMVHWK.

In another example, a fragment of SEQ ID No:3 preferably comprises an amino acid sequence of SEQ ID No: 11 (i.e. T11), i.e. HRWSSYMVHWK.

In another example, a fragment of SEQ ID No:3 preferably comprises an amino acid sequence of SEQ ID No: 12 (i.e. T12), i.e. FHRWSSYMVHWK.

In another example, a fragment of SEQ ID No:3 preferably comprises an amino acid sequence of SEQ ID No:13 (i.e. T13), i.e. EFHRWSSYMVHWK.

In other words, although detection of T14 (i.e. SEQ ID No:3) is preferred, the disclosure may also rely on detection of one or more of any of T7-T13 (i.e. SEQ ID No: 7-13).

The subject may be a vertebrate, mammal, or domestic animal. Most preferably, however, the subject is a human being, who may be male or female. The subject may be a child or adult. The age of the subject may be at least 30, 40, 50, 60, 70, 80 or 90 years old. The subject, however, may be less than 80, 70, or 60 years old. Preferably, the subject is tested before any symptoms of cognitive decline or neurodegenerative disorder are apparent. It is envisaged that the disclosure may be carried out at a similar time and in a similar manner to the way body mass index (BMI) measurements are taken.

Preferably, the method is carried out *in vitro.* Preferably, the sample comprises a biological sample. The sample may be any material that is obtainable from the subject from which protein is obtainable. Furthermore, the sample may be blood, plasma, serum, spinal fluid, urine, sweat, saliva, tears, breast aspirate, prostate fluid, seminal fluid, vaginal fluid, stool, cervical scraping, cytes, amniotic fluid, intraocular fluid, mucous, moisture in breath, animal tissue, cell lysates, tumour tissue, hair, skin, buccal scrapings, lymph, interstitial fluid, nails, bone marrow, cartilage, prions, bone powder, ear wax, or combinations thereof.

Preferably, however, the sample comprises blood, urine, tissue etc. Most preferably, the sample comprises a blood sample. The blood may comprise venous or arterial blood.

The kit may comprise a sample collection container for receiving the extracted sample.

Blood samples may be assayed for T14 levels immediately. Alternatively, the blood sample may be stored at low temperatures, for example in a fridge or even frozen before the T14 assay is conducted. Detection of T14 may be carried out on whole blood.

Preferably, however, the blood sample comprises blood serum. Preferably, the blood sample comprises blood plasma.

The blood may be further processed before the T14 assay is performed. For instance, an anticoagulant, such as citrate (such as sodium citrate), hirudin, heparin, PPACK, or sodium fluoride may be added. Thus, the sample collection container may contain an anticoagulant in order to prevent the blood sample from clotting. Alternatively, the blood sample may be centrifuged or filtered to prepare a plasma or serum fraction, which may be used for analysis. Hence, it is preferred that the T14 is analysed or assayed in a blood plasma or a blood serum sample. It is especially preferred that T14 concentration is measured *in vitro* from a blood serum sample or a plasma sample taken from the subject.

Preferably, the kit or method is used to identify the presence or absence of T14-positive cells (i.e. cells comprising SEQ ID No:3) in the sample, or determine the concentration thereof in the sample, preferably the concentration of soluble T14 and/or aggregated T14. The means for determining the T14 concentration, be it soluble or aggregated T14, may comprise an assay adapted to detect the presence and/or absence of T14-positive cells in the sample. The kit or method may comprise the use of a positive control and/or a negative control against which the assay may be compared.

T14 peptide (SEQ ID No: 3) may be assayed by a number of ways known to one skilled in the art. For example, preferably an immunoassay is employed to measure T14 peptide levels. However, it will be appreciated that non-immuno based assays may be employed, for example, labelling a compound having affinity with a ligand of the T14 peptide molecule, and then assaying for the label. T14 peptide may also be determined with Western Blot analysis, which may be used to determine the total protein level of T14 peptide. T14 peptide concentration may therefore be detected by enzyme-linked immunosorbent assay (ELISA), fluorometric assay, chemiluminescent assay, or radioimmunoassay analyses.

In one exampleof the disclosure, the concentration of (i) a soluble peptide comprising or consisting of SEQ ID No:3, or a variant or fragment thereof, or of (ii) an aggregated peptide comprising or consisting of SEQ ID No:3, or a variant or fragment thereof, is determined. In a preferred example, the concentration of (i) a soluble peptide comprising or consisting of SEQ ID No:3, or a variant or fragment thereof, and of (ii) an aggregated peptide comprising or consisting of SEQ ID No:3, or a variant or fragment thereof, is determined.

As described in Example 1, an immunoassay, such as ELISA, is most preferably used to detect soluble T14 peptide. However, as discussed in Example 2, Western Blot analysis is most preferably used to detect aggregated T14 peptide. As described in Example 4, it is preferred that both soluble and aggregated T14 can be detected in combination. Although the inventors do not wish to be bound by hypothesis, they have observed a 2kDa band and also a 50kDa band on their Western blots. Their aggregation experiments do not account for the difference in molecular weight, because a change in molecular weight was not observed when triggering aggregation in laboratory conditions. It is postulated, though unverified, that T14 peptide may be bound to a higher molecular weight protein, and that this interaction is not disrupted by the denaturing and reducing conditions of the Western Blot.

The means for determining, in the sample obtained from the test subject, the concentration of (i) a soluble T14 and/or (ii) an aggregated T14 may comprise an anti-T14 antibody or antigen-binding fragment thereof, i.e. a T14-neutralising antibody. The antibody or antigen-binding fragment thereof may be polyclonal or monoclonal. The antibody or antigen-binding fragment thereof may be generated in a rabbit, mouse or rat.

Preferably, the antibody or antigen-binding fragment thereof specifically binds to SEQ ID No:3. Preferably, the antibody or antigen-binding fragment thereof specifically binds to one or more amino acid in the C-terminus of SEQ ID No:3. Preferably, the antibody or antigen-binding fragment thereof specifically binds to one or more amino acid in SEQ ID No: 5 (i.e. SYMVHWK, which are the C-terminal amino acids numbers 7-14 of SEQ ID No: 3). Preferably, the antibody or antigen-binding fragment thereof specifically binds to a C-terminal lysine (K) residue in the epitope. The inventors have surprisingly observed that the C-terminal amino acid sequence VHWK in SEQ ID No:3, which is described herein as SEQ ID No. 6 (i.e. the C-terminal amino acids numbers 10-14 of SEQ ID No.3), acts as an epitope for the antibody or antigen-binding fragment thereof. Accordingly, more preferably the antibody or antigen-binding fragment thereof specifically binds to one or more amino acid in SEQ ID No.6. Most preferably, the antibody or antigen-binding fragment thereof specifically binds to SEQ ID No.6. Hence, it will be appreciated that the epitope to which the antibody binds comprises or consists of SEQ ID No: 6. Thus, the antibody or antigen-binding fragment thereof binds specifically to SEQ ID No:3, or a fragment or variant thereof, and can be used as or in the detection means.

Preferably, the antibody or antigen-binding fragment thereof does not bind to SEQ ID No:2 (i.e. T30).

Preferably, the antibody or antigen-binding fragment thereof does not bind to SEQ ID No:4 (i.e. T15), i.e. NQFDHYSKQDRCSDL.

Preferably, the antibody or antigen-binding fragment thereof does not bind to SEQ ID No: 14 (i.e. β-amyloid (Aβ), i.e.
DAEFRHDSGYEVHHQKLVFFAEDVGSNKGAIIGLMVGGVVIA.

The kit of the second example may further comprise a label which may be detected. The term "label" can mean a moiety that can be attached to the means for determining, in the sample obtained from the test subject, the concentration of (i) a soluble T14 and/or (ii) an aggregated T14. Moieties can be used, for example, for therapeutic or diagnostic procedures. Therapeutic labels include, for example, moieties that can be attached to the antibody or fragment thereof described herein, and used to monitor the binding of the antibody to the T14 peptide (i.e. SEQ ID No:3, or fragment or variant thereof). Diagnostic labels include, for example, moieties which can be detected by analytical methods. Analytical methods include, for example, qualitative and quantitative procedures. Qualitative analytical methods include, for example, immunohistochemistry and indirect immunofluorescence. Quantitative analytical methods include, for example, immunoaffinity procedures such as radioimmunoassay, ELISA or FACS analysis. Analytical methods also include both in vitro and in vivo imaging procedures. Specific examples of diagnostic labels that can be detected by analytical means include enzymes, radioisotopes, fluorochromes, chemiluminescent markers, and biotin.

Preferably, soluble T14 peptide serum or T14 peptide plasma concentrations may be measured by double-antibody sandwich ELISA, which will be known to the skilled technician. The ELISA may comprise using a suitable antibody for coating a microtiter plate. For example, such a suitable antibody may comprise an affinity-purified polyclonal anti- T14 peptide antibody described herein (WO 2016/156803). Furthermore, the ELISA may comprise using a suitable antibody for detection. For example, such a suitable antibody may comprise peroxidase-labelled monoclonal mouse anti human T14 peptide antibody. Human T14 peptide, which may be purified from plasma, and which then may be quantified by amino acid analysis, may be used to calibrate a plasma standard using standard techniques known to the skilled technician. A label can be attached directly to the antibody, or be attached to the secondary binding agent that specifically binds T14. Such a secondary binding agent can be, for example, a secondary antibody. A secondary antibody can be either polyclonal or monoclonal, and of human, rodent or chimeric origin.

Examples 1 and 2 describe ELISA and Western Blot data for T14 in a range of healthy individuals, as well as in patients suffering from Alzheimer's disease. It will be appreciated that the reference value of T14 peptide, be that in soluble or aggregated form, in an individual (healthy or AD) is highly dependent on a number of factors, for example, their age, whether or not the subject is suffering from a neurodegenerative and, if so, how far that disorder has progressed.

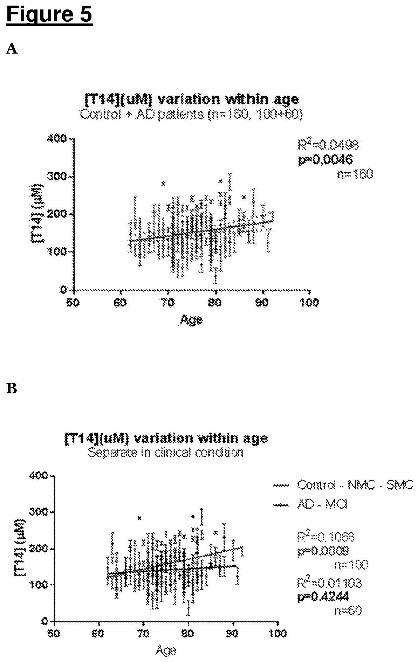

Referring to Figure 5A, there is shown the correlation of T14 concentration with age for the entire cohort (n = 160), and Figure 5B shows the correlation of T14 concentration with age for amyloid negative, i.e. Control group less those subjects showing No Memory Complaints less those subjects showing Subjective Memory Complaints (n=100) and amyloid positive, i.e. Alzheimer's Disease less those subjects showing mild cognitive impairment. As can be seen, in Figure 5B, T14 levels increase for the control group with age (green line), whereas there is little change in T14 concentration of Alzheimer's patients (black line). Furthermore, as the population gets older, the degree of separation between respective T14 concentrations becomes much more pronounced, and so it is clear that age plays a factor on T14 levels. As such, the inventors believe that it is easier to distinguish between healthy subjects (green line) and Alzheimer's patients (black line) for older subjects. However, clearly, there must be a trade off so that the methods of the invention are employed before a test subject begins to show any symptoms of cognitive decline, i.e. it has been left too late.

Hence, the reference value described herein is preferably calculated from a plurality of individuals who are between 60 and 90 years old, and most preferably between 70 and 80 years old, some of whom are healthy and some of whom are showing at least some cognitive decline. The reference value may be produced by designating members of the reference population as either Amlyoid positive or Amyloid negative. In one embodiment, such designation may be achieved by assessing beta-amyloid relative values on the BeCKeT scale. For example, an Amyloid negative patient may have a BeCKeT value of <1.4, and an Amyloid positive subject may have a BeCKeT value of >1.4.

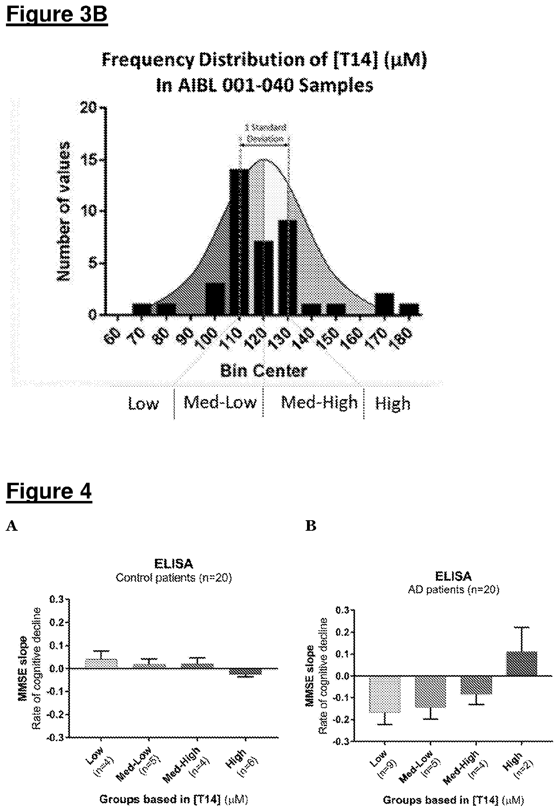

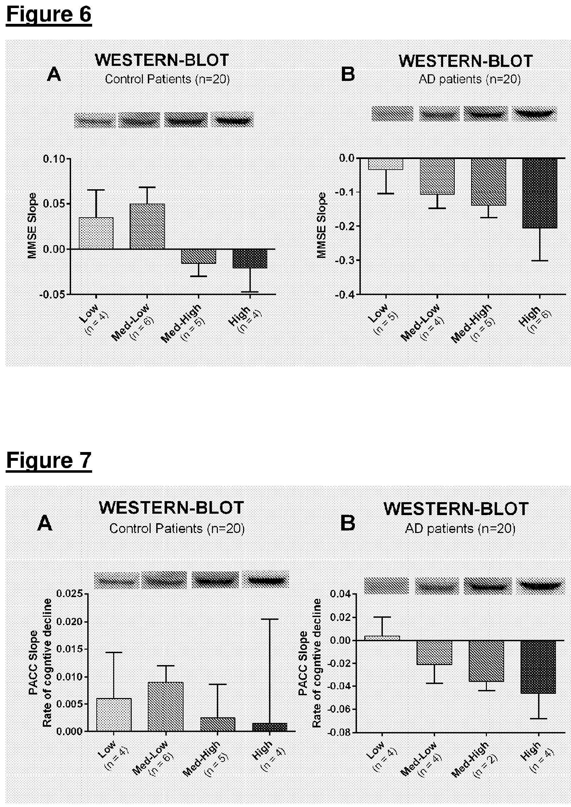

With reference to Figure 4B, the inventors have shown that, when ELISA is used to detect soluble T14 concentrations in Alzheimer's patients, surprisingly lower T14 concentrations correlate with a faster rate of cognitive decline than those patients having higher T14 concentrations. With reference to Figures 6B and 7B, the inventors were also surprised to observe that when Western Blotting is used to detect aggregated T14 concentration in Alzheimer's patients, higher T14 concentrations correlate with a faster rate of cognitive decline than those patients having lower T14 concentrations. Accordingly, measurement of soluble or aggregated T14 concentrations gives the opposite results when correlating to the rate of cognitive decline. Due to current limitations of the assays (both western blot and ELISA), all values in both cases can currently only be considered relative to different groups. Although the inventors do not wish to be bound by hypothesis, they believe that since Western Blots measure aggregates of the T14 peptide, these data suggest that the aggregated T14 peptide, or it's conjugation with an unknown protein or carrier protein present in plasma plays a role in neurodegeneration. Furthermore, since ELISA measures soluble T14, the inventors hypothesise that there could be free monomer that it would be measuring has been taken up and/or is bound to its respective targets depleting the circulating pool of monomer (or soluble/unbound) T14 peptide.

The inventors have noted that the concentration of soluble T14 in the test subjects was statistically less than the reference concentration, as calculated using the method described in the Example. This is referred to herein as the `lowered' concentration of soluble peptide comprising or consisting of SEQ ID No:3, or a variant or fragment thereof. Conversely, the inventors show that the concentration of aggregated T14 in the test subjects was statistically higher than the reference concentration, as calculated using the method described in the Example. This is referred to herein as the 'raised' concentration of aggregated peptide comprising or consisting of SEQ ID No:3, or a variant or fragment thereof.

The skilled technician will appreciate how to measure the concentrations of T14 peptide (either soluble or aggregated) in a statistically significant number of control individuals, and the concentration of T14 in the test subject, and then use these respective figures to determine whether the test subject has a statistically significant increase or decrease in T14 concentration, and therefore infer whether that subject is at risk from developing in later life a higher rate of cognitive decline.

Accordingly, the inventors have realised that the difference in concentrations of T14 between the normal and raised/lowered levels, for aggregated or soluble T14, respectively, can be used as a physiological marker, suggestive of the likelihood for developing a higher rate of cognitive decline than would normally be the case for that individual. It will be appreciated that if a subject has a lowered concentration of soluble T14 which is considerably lower than the reference soluble T14 concentration, or a raised concentration of aggregated T14 which considerably high than the reference aggregated T14 concentration, then they would be at a higher risk of suffering from a higher rate of cognitive decline than if the concentration of T14 was only marginally higher or lower than the reference T14 concentrations.

By way of example, the decrease in concentration of soluble T14 from the reference concentration may be approximately 10%, preferably about a 20% decrease, more preferably about a 30% decrease, even more preferably about a 40% decrease, and most preferably a decrease of about 50% from the reference concentration. Such decreases in soluble T14 concentrations infer that the test subject will suffer from a higher rate of cognitive decline. Alternatively, the increase in concentration of aggregated T14 from the reference concentration may be approximately 10%, preferably about a 20% increase, more preferably about a 30% increase, even more preferably about a 40% increase, and most preferably an increase of about 50% from the reference concentration. Such increases in aggregated T14 concentrations infer that the test subject will suffer from a higher rate of cognitive decline. Accordingly, a clinician would be able to make a decision as to the preferred course of treatment required, for example, the type and dosage of the therapeutic agent according to the third example to be administered.

The methods of the invention may comprise measuring the rate of cognitive decline by a Mini Mental State Examination (MMSE) score or a Preclinical Alzheimer Cognitive Composite (PACC) score.

MMSE is a questionnaire that is administered virtually universally in those with suspected AD, and in broader study cohorts, as a measure of cognitive impairment. It is widely considered a gold standard for diagnosis in AD due to its ease of application with little training required, repeatability, validity and reliability. It is also particularly useful when considering the longitudinal assessment of AD and its progression. Repeated measures of MMSE score, collected at regular intervals, may be used to calculate a rate of cognitive decline for the subject. Then, a linear regression is preferably performed to calculate the slope of MMSE score change over time, and it is this slope which is interpreted as the rate of cognitive change (decline/incline). Preferably, therefore, cognitive decline is measured in terms of MMSE score. The slope may be calculated in points dropped on the MMSE score per month, for example low T14 levels in Figure 4B correlate with a drop in MMSE score of 0.15 points per month.

The PACC test, on the other hand, combines tests that assess episodic memory, timed executive function, and global cognition. It is the primary outcome measure for the first clinical trial in preclinical AD. In Figure 7B, for AD patients, high T14 levels are associated with faster decline.

Preferably, the higher rate of cognitive decline equates to at least 5%, at least 10% or at least 15% cognitive decline greater than that seen in the reference value. More preferably, the higher rate of cognitive decline equates to at least 20%, at least 25% or at least 30% cognitive decline greater than that seen in the reference value.

Preferably, the method comprises a step of age-adjusting the T14 concentrations from the test subject, be they soluble T14 or aggregated T14, against the corresponding reference value.

Preferably, the method comprises a step of detecting the presence or absence of beta-amyloid in the sample from the test subject. For example, detection may be performed using beta-amyloid imaging, such as PET imaging. The method may comprise a step of designating the test subject as either Amyloid positive or Amyloid negative. In one embodiment, such designation may be achieved by assessing beta-amyloid relative values on the BeCKeT scale. For example, an Amyloid negative patient may have a BeCKeT value of <1.4, and an Amyloid positive subject may have a BeCKeT value of >1.4.

Most preferably, the method comprises the use of a combination of age-adjusted T14 levels and amyloid beta imaging to predict the likelihood of a higher rate of cognitive decline. The steps of the method may be conducted in any order. For example, in one embodiment, the method may comprise determining age-adjusted deviation in T14 concentrations, followed by determining the rate of cognitive decline, followed by detection of beta-amyloid.

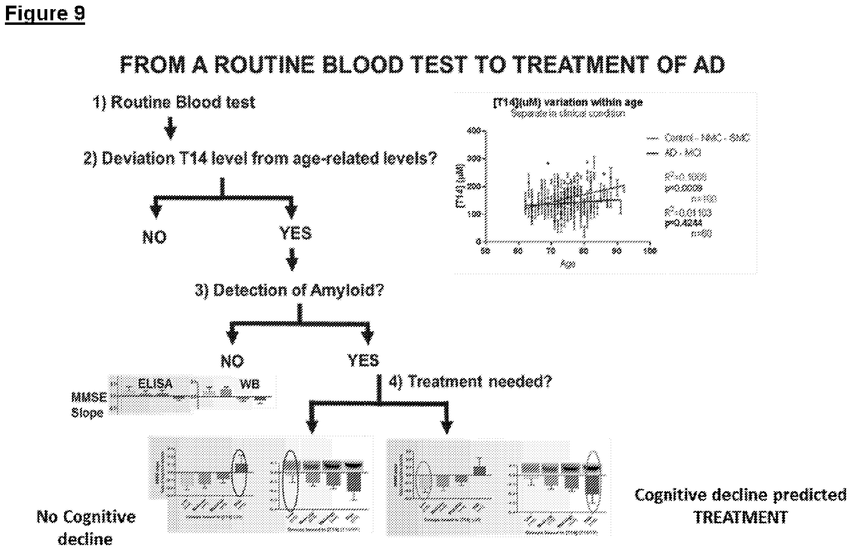

As shown in Figure 9 and Example 3, the inventors have devised a novel prognostic method according to the first example for initially detecting, and subsequently treating neurodegenerative disorders, such as Alzheimer's disease, according to the third example.

Preferably, a first step of the method comprises conducting a blood test on a sample taken from the subject, for example by their doctor or nurse, wherein soluble and/or aggregated T14 concentrations are detected using ELISA and/or Western blot.

Preferably, a second step of the method comprises comparing the measured T14 levels against expected age-related reference concentrations. For example, the Western Blot or ELISA would include age-matched reference samples from control and/or diseased subjects, or certified reference materials to determine relative levels of T14. In an embodiment in which little or no deviation is detected with respect to the age-related reference concentrations (i.e. there is little or no separation between the green and black lines shown in Figure 5B), the subject may be deemed to be healthy with little risk of developing a higher rate of cognitive decline. However, in an embodiment in which deviation detected (i.e. there is a statistically significant separation between the green and black lines shown in Figure 5B), then a third step of the method preferably comprises detection of amyloid, for example using an amyloid PET scan. If no amyloid is detected, then the subject may be deemed to be healthy with little risk of developing a high rate of cognitive decline. However, if amyloid is detected, then a fourth step of the method preferably comprises prediction of the rate of cognitive decline. Predication of the rate of cognitive decline may comprise comparison to pre-defined limits of deviation from expected levels of T14 for a given age group.

Finally, when cognitive decline is predicted, the method preferably comprises a fifth step of therapeutic treatment.

It will be appreciated that the disclosure extends to any nucleic acid or peptide or variant, derivative or analogue thereof, which comprises substantially the amino acid or nucleic acid sequences of any of the sequences referred to herein, including functional variants or functional fragments thereof. The terms "substantially the amino acid/nucleotide/peptide sequence", "functional variant" and "functional fragment", can be a sequence that has at least 40% sequence identity with the amino acid/nucleotide/peptide sequences of any one of the sequences referred to herein, for example 40% identity with the sequence identified herein.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (v) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22,4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)^{∗}100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula:-Sequence Identity = (N/T)^{∗}100.

Alternative methods for identifying similar sequences will be known to those skilled in the art. For example, a substantially similar nucleotide sequence will be encoded by a sequence, which hybridizes to DNA sequences or their complements under stringent conditions. By stringent conditions, we mean the nucleotide hybridises to filter-bound DNA or RNA in 3× sodium chloride/sodium citrate (SSC) at approximately 45°C followed by at least one wash in 0.2× SSC/0.1% SDS at approximately 20-65°C. Alternatively, a substantially similar polypeptide may differ by at least 1, but less than 5, 10, 20, 50 or 100 amino acids from the sequences shown in SEQ ID No: 1-14.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the disclosure, and to show how embodiments of the same may be carried into effect, reference will now be made, by way of example, to the accompanying Figures, in which:-
**Figures 1-9** show data generated using 160 samples from an Australian Imaging Biomarker & Lifestyle (AIBL) study. **Figure 1** shows Receiver Operating Characteristic (ROC) curves which employ multiple logistic regression to model the likelihood (log odds) of cognitive impairment across the range of T14 'cut points'. These ROC curves were used to assess the value of the WB and ELISA assays;
**Figure 2** shows MMSE scores in relation to disease progression;
**Figure 3A** shows the frequency distribution of T14 levels in the AIBL study for all 160 samples as measured by ELISA (µM T14), and the criteria for selection of groups (Low, Medium-Low, Medium-High and High groups) as determined by relative values of T14, in control, no memory complaints (NMC), subjective memory complaints (SMC), Alzheimer's disease (AD) and mild cognitive impairment (MCI) subjects. **Figure 3B** shows the frequency distribution of T14 levels for an initial 40 samples that were originally obtained from AIBL as part of a pilot study;
**Figure 4A** shows the correlation of T14 with severity of cognitive decline (as measured by mini-mental state examination, MMSE) in ELISA for the cohort subset of 20 control AIBL study patients (Amyloid negative patients, BeCKeT <1.4). **Figure 4B** shows the same analysis for the 20 AD/MCI patient subset (Amyloid positive, BeCKeT >1.4). The more negative MMSE slope indicates quicker progression of cognitive decline;
**Figure 5A** shows the correlation of T14 with age for the entire AIBL Study cohort (n = 160), and **Figure 5B** for amyloid -ve (Control-NMC [No Memory Complaints]-SMC [Subjective Memory Complaints], n=100) and amyloid +ve (AD-MCI [mild cognitive impairment]);
**Figure 6A** shows the correlation of T14 with severity of cognitive decline (as measured by mini-mental state examination, MMSE) in Western Blot for the 20 control AIBL study patients (Amyloid negative patients, BeCKeT < 1.4), and **Figure 6B** shows the correlation of T14 with severity of cognitive decline (as measured by MMSE) in Western Blot for the 20 AD/MCI patient subset (Amyloid positive, BeCKeT >1.4). The more negative MMSE slope indicates quicker progression of cognitive decline;
**Figure 7A** shows the correlation of T14 with severity of cognitive decline (as measured by PACC) in Western Blot for the 20 control AIBL study patients (Amyloid negative patients, BeCKeT <1.4), and **Figure 7B** shows the correlation of T14 with severity of cognitive decline (as measured by MMSE) in Western Blot for the 20 AD/MCI patient subset (Amyloid positive, BeCKeT >1.4). The more negative MMSE slope indicates quicker progression of cognitive decline;
**Figure 8** shows the correlation between T14 levels with MMSE score for the entire AIBL cohort, separated into above MMSE score 26 for A) aggregate T14, B) soluble T14; between MMSE score 20-25 for C) aggregate T14, D) soluble T14; below MMSE score 20 for E) aggregate T14, F) soluble T14; recorded at the time of blood collection. Yellow highlights indicate statistical significance;
**Figure 9** is a model of a potential routine blood test from T14 quantification to cognitive decline prediction;
**Figure 10** shows the WB data generated from the analysis of samples from NeurosScios Clinic, Austria, and shows the correlation between change in T14 value and rate of MMSE change in 22 AD probable patients from the Austrian cohort;
**Figures 11-33** show data generated using a pooled database with patients from AIBL, Sweden and Austria. Analysis involving age only includes data from the AIBL and Austrian cohort, and analysis involving MMSE includes data from AIBL, Austrian and Swedish cohort. **Figure 11** shows a box and whisker plot of databank sources. Box = 25-75 percentile, diamond = mean, horizontal line = median. Groups appear reasonably comparable;
**Figure 12** shows a box and whisker plot of Dementia groups, which appear reasonably comparable;
**Figure 13** shows a box and whisker plot of Age groups. There are little differences in the distributions. T14 appears to increase with age but with variability increasing with age as well;
**Figure 14** shows a box and whisker plot of MMSE groups. There appear to be little distributions;
**Figure 15** shows a correlation plot of AD Subjects: Correlation T14 vs Age;
**Figure 16** shows a correlation plot of Normal Subjects: Correlation T14 vs Age;
**Figure 17** shows a correlation plot of AD Subjects: Correlation T14 vs MMSE;
**Figure 18** shows a correlation plot of Normal Subjects: Correlation T14 vs MMSE;
**Figure 19** shows ANCOVA Model Fit T14 vs Age By Dementia Group. This graph may indicate a consistent quantitative difference in T14 between Dementia groups (almost parallel) at difference ages. T14 values are higher in the Normal group than in the AD group. T14 values overall increase with Age;
**Figure 20** shows ANCOVA Model Fit T14 vs MMSE By Dementia Group. This graph may indicate quantitative differences between Groups with MMSE score picked up in ANCOVA model statistics - T14 values are higher in the Normal group. T14 values are overall lower with higher MMSE;
**Figure 21** shows Overall Group Differences: Adjusted Means (+/-95% CI). Adjusted mean difference (95%CI) between AD-Control = -0.10 (-1.8 to -0.02). (The ratio of AD/Control expressed as a percentage was 90.0% (84.4% to 96.0%) representing a difference between the two groups of 10%). Consistent differences were noted with log transformed T14 using log ANCOVA and also using CMH test. Significant differences were notable between AD-Control for the 70-79 age group;
**Figure 22** shows Group Differences according To Age: Adjusted Means (+/-95% CI);
**Figure 23** shows Distribution: AD vs Normal Classified Subjects - Overall;
**Figure 24** shows ROC Analysis: AD vs Normal Classified Subjects - Overall. Using Youden J maximum (J=1.17) to identify the maximum accuracy the sensitivity was 64.2% and specificity 53.1% producing a diagnostic 'cut point of 1.008;
**Figure 25** shows ROC Analysis: AD vs Normal Classified Subjects - Overall With Age. NB: AUC increased from 0.58 to 0.63;
**Figure 26** shows Distribution: AD vs Normal Classified Subjects - Over 50 yrs;
**Figure 27** shows ROC Analysis: AD vs Normal Classified Subjects - Over **50** yrs;
**Figure 28** shows Distribution: AD vs Normal Classified Subjects - Over 60 yrs;
**Figure 29** shows ROC Analysis: AD vs Normal Classified Subjects - Over 60 yrs;
**Figure 30** shows Distribution: AD vs Normal Classified Subjects - Over 70 yrs;
**Figure 31** shows ROC Analysis: AD vs Normal Classified Subjects - Over 70 yrs;
**Figure 32** shows Distribution: AD vs Normal Classified Subjects - Over 80 yrs;
**Figure 33** shows ROC Analysis: AD vs Normal Classified Subjects - Over 80 yrs; and
**Figure 34** shows a barchart of Lysine (K) capped C-terminus 5-14mer variants of T14, i.e. SEQ ID No:3 (150nM conc.).

### Examples

Since both the incidence and prevalence of AD increases with age, the number of patients is growing significantly with an aging population. Unfortunately, there currently exists no single test that can be administered to diagnose Alzheimer's Disease, and its clinical classification relies on a combination of subjective reporting, medical history evaluation, cognitive function tests, and costly brain imaging scans with no true classification possible until a post-mortem examination of the brain can be conducted. The identification of a biomarker that allows detection of AD with a high degree of specificity and sensitivity from a non-invasive, easily repeated, cost-effective procedure such as blood collection would enable routine screening, early intervention, and potentially revolutionise neuropathological research by opening the pathway for new strategies in intervention.

The inventors present herein a peptide derived from the C-terminus of acetylcholinesterase (AChE), which is present as a naturally occurring bioactive molecule in brain tissue. This peptide is called T14 (SEQ ID No: 3). WO 2016/156803 describes an antibody raised against this C-terminus peptide can selectively recognise and quantify this innate signalling molecule in either human and rat brain tissue by an indirect ELISA immunoassay or Western blotting technique. Now, employing the same procedures, the inventors have correlated varying T14 levels with age, and show that it can be used as a quantitative predictor or biomarker of the rate of cognitive decline. They have also demonstrated that fragments of T14 (e.g. T7-T13) can also be detected and used as a robust biomarker. The inventors assessed T14 using plasma from three separate sources: 1) Australian Imaging Biomarker & Lifestyle (AIBL). 2) NeurosScios Clinic, Austria. 3) Biobank Sverige, Sweden.

### Example 1 - AIBL cohort: Detection of T14 peptide in sample using an antibody in ELISA

Using ELISA, which detects soluble T14, the inventors have analysed human plasma samples correlating varying T14 concentrations with age.

### Materials and Methods

### ELISA for T14 peptide antibody

The standard curves and the samples were run in triplicate. The plasma samples were diluted 1:10,000 in PBS Buffer. The standard curve for determination of T14 peptide in brain tissue samples were diluted in PBS buffer. The standard curve ranged from 8 to 100 nM of T14, and the blank was PBS buffer alone. Briefly, 96-well immunoplates (NUNC) were coated with 100µl/well of sample or standard T14, covered with parafilm and incubated overnight at 4 °C. The following day the sample was removed by flicking the plate over a sink with running water, and 200 µl of the blocking solution containing 2% bovine serum albumin (BSA) in Tris-buffered saline and Tween 20 (TBS-T) was added and incubated for 4h at room temperature. Blocking solution was then removed and 100 µl of antibody (described in WO 2016/156803), diluted in blocking solution to 1µg/ml, was added and incubated overnight at 4 °C. The primary antibody was removed the next day and wells were washed 3 times with 200 µl of TBS-T. After 100 µl of secondary enzyme-conjugated antibody (see WO 2016/156803) diluted in blocking solution to 0.1 µg/ml was added and incubated for 2 h at room temperature; the plate was covered with parafilm during all incubations. After 2 h, the plate was washed 4 times with TBS-T. The addition of 3,3,5,5-tetramethylbenzidine started the colour reaction. The reaction was stopped 20 min later with stopping solution containing 2 M H2SO4, and the absorbance was measured at 450 nm in a Versamax plate reader (Molecular Devices, Wokingham, UK).

### Calculation of cognitive decline through longitudinal MMSE data

Repeated measures of MMSE score, as collected by the AIBL study at 18 month intervals, were used to calculate a rate of cognitive decline for each individual patient. Briefly, a linear regression was performed to calculate the slope of MMSE score change over time. This slope is interpreted as the rate of cognitive change (decline/incline).

### Results and Discussion for ELISA

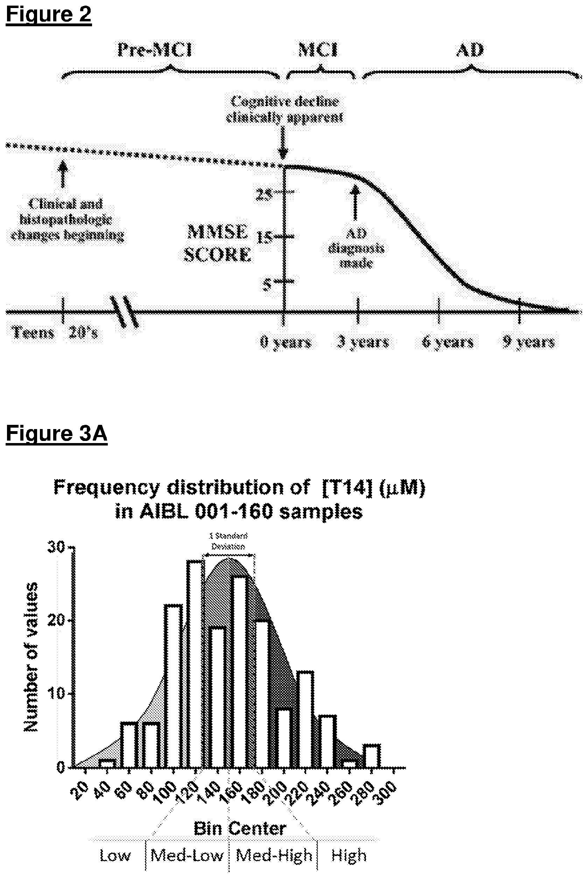

The inventors obtained an initial cohort of 40 patients, and then a second cohort of 160 patients provided by the Australian Imaging Biomarker & Lifestyle (AIBL). Referring to Figure 3 (upper graph), the analysis of the full cohort of 160 patients provided by the AIBL study shows that the distribution of values in the large cohort, determined by calculation of T14 content, as derived from a standard curve of the exogenous peptide in PBS, follows a normal distribution (D'Agostino & Pearson omnibus normality test P=0.0929). This Bell curve distribution enables the opportunity for the analysis of the samples to be based on a number of discrete groups that are described by their distribution around the mean.

All samples that are within the standard deviation of the mean are considered to be in the "medium" group (medium levels of T14). All samples below the mean are considered to be in the "low" T14 group, while all above the mean are considered to be in the "high" T14 group. These descriptions apply to those values that are within the medium group (within the standard deviation of the mean) and also extend beyond this group. Using this method, the inventors have defined four discrete groups: Low, Med-Low, Med-High, and High, based on the distribution of values.

The normality of the distribution holds true for both cohorts when taken as a single whole (see Figure 3A), and for the original cohort of 40 patients (see Figure 3B).

AIBL samples were supplied in two cohorts with a first cohort containing 40 patients (20 control, 2 MCI, 18 AD) and the second cohort containing 120 patients. In the first cohort of 40 patients, the clinical classification of each subject is determined by their BeCKeT score derived from amyloid imaging scans. All subjects with a BeCKeT score <1.4 are defined as "Control", and all subjects with a BeCKeT score >1.4 are defined as "AD". Referring to Figure 4A, there are shown the results from the ELISA analysis of control subjects. The control subjects show no trend associated with levels of T14.

Figure 4B, however, shows the same analysis for those subjects clinically classified as AD/MCI (Mild cognitive impairment). This subset of patients show a trend which suggests that lower levels of T14 are associated with a faster rate of cognitive decline than those with higher levels of T14. The trend appears to be continuous between the groups with each group, in increasing levels of T14, showing less severe cognitive decline than the group immediately below.

Referring to Figure 5A, regression analysis of the entire cohort of 160 subjects comparing levels of T14 with age shows a significant positive correlation with age. When the cohorts are divided by their clinical status (as determined by levels of amyloid beta/BeCKeT score), as shown in Figure 5B, the controls (including NMC and SMC) retain a significant positive correlation with age (R² = 0.1068, p=0.0009), while the AD/MCI portion of the cohort show no significant correlation with age (R² = 0.01103, p=0.4244). This separation of the cohorts based on age may offer age-related parameters of risk of cognitive decline to be determined with a more discriminating serological test.

### Example 2 - AIBL cohort: Detection of T14 peptide in sample using an antibody in Western Blot

Given that T14 is detectable at several different molecular weights within the range of the western blot when probing western blot membranes with the T14 antibody, the band at 50kDa was selected for analysis as it has the most dynamic range of signal modulation. The aim of this experiment was to assess, using Western blotting, how the endogenous T14 species reflects AD pathology and its potential as an AD biomarker. Peptide aggregate levels were initially measured in human brain areas and CSF to establish peptide CNS behaviour. Most importantly, peptide aggregate levels in blood plasma were measured using Western blotting, and this was subsequently linked to patient cognitive function to demonstrate a correlative relationship.

### Materials and methods

### Measuring protein sample concentration

Protein concentrations from the above samples were measured using the Pierce^{™} 660nm Protein Assay (Thermo Scientific). In short, a serial dilution (0 to 2 mg/ml) was made from a 10 mg/ml stock of bovine serum albumin (BSA). Three replicates of each BSA concentration were prepared by transferring 10 µl of the protein into a clear 96 well plate (Greiner). Then, samples were diluted with three concentrations (1:1, 1:2, 1:10) and three replicates of each concentration were placed into the same 96 well plate with each replicate containing 10 µl of sample. Subsequently, 150 µl of Pearce Reagent was added to the standards and all samples and the mixture was left to incubate for 5 min with gentle shaking. Finally, the plate was read on a spectrophotometer (Molecular Devices) at 660 nm. The protein concentrations of the samples were determined using the slope and y-intercept from the BSA standard curve, both calculated via Microsoft Excel.

### Polyacrylamide gel electrophoresis of protein samples

Polyacrylamide gels (mini- PROTEAN ^{®} TGX stain free^{™} gels, BIO-RAD) were placed into the electrophoresis tank (BIO-RAD, mini-PROTEAN tetra system) and Running buffer (25 mM TRIS-base, pH 8.6, 192 mM glycine, 0.1% SDS) was added the gel and tank reservoirs (BioRad). Protein samples were prepared by mixing with distilled water and 4× Laemmli sample buffer (final concentrations: 69.5 mM TRIS-HCl pH 6.8, 1.1% LDS, 11.1% (w/v) glycerol, 0.005% bromophenol blue, BIO-RAD) and 2.5% mercaptoethanol (BIO-RAD). The mixtures were heated at 95°C for 5 min before cooling on ice. Samples and the positive control were loaded into the gels and were electrophoresed alongside with a molecular weight marker (Precision Plus Protein^{™} Dual Xtra Standards, BIO-RAD) at 35mV for 90 min. Ice block was placed inside the running tank to prevent any overheating.

### Transfer of protein samples onto PVDF membrane

Stacking gels were trimmed off and the separating gels were transferred onto PVDF Transfer Membrane (Thermo Scientific) in a Mini Transblot Cell (BIO-RAD). Briefly, the PVDF Transfer Membrane was activated by soaking with methanol for 1 min followed by soaking with distilled water for 2 min. All layers were subsequently saturated with transfer buffer (20 mM TRIS-base pH 8.6, 154 mM glycine, 0.8% w/v SDS and 20% methanol). The transfer sandwich, in the order of bottom to top, consists of transfer sponge, blotting paper, the gel, PVDF Transfer Membrane, blotting paper, transfer sponge were placed into a transfer cassette, which was inserted into the Mini Transblot Cell filled with Transfer Buffer. Finally, electrophoretic transfer took place for 90 min at 200mA. Ice block was placed inside the transfer tank to prevent any overheating.

### Staining of PVDF membrane

BLOT-Faststain^{™} (G-Biosciences, USA) was used to stain for total protein, acting as the loading control. Immediately after electrophoretic transfer, the PVDF transfer membrane was stained with the diluted BLOT-Faststain^{™} fixer solution (10 fold) for 2 min with gentle shaking. The membrane was then incubated with the diluted BLOT-Faststain^{™} developer solution (4 fold) for 1 min with gentle shaking. Subsequently, the membrane was stored at 4°C in the dark in the developer solution for 30 min to allow protein bands to reach maximum intensity. Finally, the membrane was washed with cold water to eliminate background staining and imaged using the G box (Syngene). The membrane can then be destained using warm deionised water (40-45°C) and ready for the blocking stage.

### Detection of protein bands

The PVDF transfer membrane was blocked in TBS (TRIS-buffered saline, 20 mM TRIS-base pH 7.5, 0.5 mM NaCl) containing 5% skimmed milk powder for 1 h, then washed twice for 7 min each in TTBS (TBS supplemented with 0.05% v/v Tween-20). The membrane was incubated overnight at 4°C with a primary antibody diluted in TTBS containing 1% skimmed milk powder. On the following day, the primary antibody was removed. The membrane was washed three times for 5 min each in TTBS, then incubated for 1 h at room temperature with the secondary antibody. The secondary antibody of choice depends on the type of primary antibody used. It can either be goat anti mouse secondary antibody conjugated to HRP (a9309, Sigma, diluted in TTBS containing 1% skimmed milk powder (working concentration: 1:1000) or goat anti rabbit secondary antibody conjugated to HRP (ab6721, abcam) diluted in TTBS containing 1% skimmed milk powder (working concentration: 1:5000). After secondary antibody incubation, membranes were washed three times for 5 min in TTBS before a final 10 min wash in TBS. Protein bands were detected using the G box (Syngene).

**Table 1 - Primary antibodies used for Western blotting detection**

| **Antibody** | **Species Raised in** | **Company** | **Catalogue Number** | **Working cone.** |
|---|---|---|---|---|
| Anti-T14 | Rabbit | Neuro-Bio/ Genosphere | N/A | 1:1000 |
| Anti-Amyloid beta | Rabbit | Cell Signalling Technology | 8243 | 1:1000 |
| Anti-P-Tau | Mouse | Thermo Fisher | MN1020 | 1:1000 |

### Protein band imaging and data analysis

The PVDF membrane was placed in the G box (Syngene). The focus and zoom settings were adjusted to ensure that the membrane was at its largest at the centre of the screen. Luminol and Peroxide solutions from Clarity^{™} Western ECL substrate (BIO-Rad) were mixed the equal parts and applied to the membrane. Images were taken in the dark at 1 min time intervals for 5 min to obtain the optimal signal for the protein bands. Following that, the membrane was exposed in white light using an automatic setting in order to obtain an image for the molecular ladder. The blot images were then analysed using image J. Box of equal sizes were placed around protein bands in each lane, allowing measurement of protein band intensities. The background was then subtracted from the band intensities and the results were analysed in Microsoft Excel and the Graphpad software.

### Antibody stripping for re-probing

The protein signal from the PVDF transfer membrane can be stripped and reprobed for a different protein. Briefly, the membrane was washed with mild stripping buffer (200mM Glycine, 3.5mM SDS, 1% v/v Tween-20, pH 2.2) twice for 10 min each. Subsequently, the membrane was washed with PBS twice for 10 min each and then washed with TTBS twice for 5 min each. Clarity^{™} Western ECL substrate (BIO-Rad) was added to the membrane and imaged using the G Box (Syngene) in order to check for residual protein signals. If residual signal was too strong, then the whole stripping process was repeated. Then, the membrane was ready for subsequent blocking stage and primary antibody probing (see above).

### MMSE

MMSE (Mini Mental State Examination) is a questionnaire that is administered virtually universally in those with suspected AD, and in broader study cohorts, as a measure of cognitive impairment. It is widely considered a gold standard for diagnosis in AD due to its ease of application with little training required, repeatability, validity and reliability. It is also particularly useful when considering the longitudinal assessment of AD and its progression.

### Results and Discussion for Western Blot

The inventors obtained an initial cohort of 40 patients provided by the Australian Imaging Biomarker & Lifestyle (AIBL), containing 20 control, 2 MCI, 18 AD. The clinical condition was classified using amyloid PET imaging with BeCKeT score ≥ 1.4 being defined as AD.

T14 levels were measured using Western Blot, normalised to total protein (the loading control), and separated into the control and MCI/AD subjects. Subsequently, T14 values were ranked from Low to High for control and AD subjects and split into statistical quartiles such that there are four groups of values. The "low" group lies below the lower quartile, the "medium low" group lies between the lower quartile and median, the "medium high" group lies between the median and the upper quartile and the "high" group lies above the upper quartile.

Next, using longitudinal data, the rate of cognitive decline were calculated using two separate cognitive tests: MMSE and PACC. They were then pooled depending on their clinical condition and the subgroup they belong to base on their T14 values. Referring to Figure 6A, in control subjects, there is no association in T14 level and rate of decline as measured by MMSE. However in AD patients, referring to Figure 6B, lowest T14 level associated with slowest cognitive decline and the rate of decline gradually increases with increasing level of T14 with the highest T14 group displaying the fastest decline as measured by MMSE.

Another cognitive test that can measure amyloid related decline is known as PACC (Preclinical Alzheimer Cognitive Composite). PACC combines tests that assess episodic memory, timed executive function, and global cognition. It is the primary outcome measure for the first clinical trial in preclinical AD. Referring to Figure 7A, Western blot assessment of the original cohort of 40 patients showed that there is no association between T14 levels in control patients and rate of PACC decline. In Figure 7B, however, for AD patients, high T14 levels are associated with faster decline. Hence, a similar pattern to the MMSE results is shown when PACC scored were used to measure cognitive decline. Figure 7A shows that control subjects displayed no association between T14 levels and rate of cognitive decline, whereas Figure 7B demonstrated the strong association between T14 levels with rate of cognitive decline.

Rate of cognitive decline is determined as rate of change in MMSE score over time. The AIBL study encompasses data collection over 18 month intervals, with a variety of participation durations within the cohort. The slope (change in MMSE) was calculated for each individual case. More negative slopes indicate a more rapid cognitive decline over the course of the study, while a positive slope indicates an increase in cognitive capacity over the course of the study.

As the clinical classifications in this cohort are determined by their relative values on the BeCKeT scale, it follows that amyloid imaging could play a complimentary role in predictability of cognitive decline, in conjunction with determination of circulating levels of T14 in plasma.

### Example 3 - AIBL cohort: Development of a blood test to detect and treat Alzheimer's disease

Pulling together their conclusions based on the data discussed in Examples 1 and 2, the inventors have developed a novel medical test to detect and treat Alzheimer's, and a schematic is shown in Figure 9. The model uses a combination of age-adjusted T14 levels and amyloid beta imaging to predict the likely rate of cognitive decline that a patient will develop, as follows:-
1) Step 1 - A blood test is taken from the subject (for example by their doctor or nurse), and soluble and/or aggregated T14 concentrations are then detected using ELISA and/or Western blot. As shown by ELISA, soluble T14 levels displayed age dependency for control subjects, but not for AD patients;
2) Step 2 - The measured T14 levels are then compared to expected age-related concentrations, for example by comparing the degree of separation between the green and black lines shown in Figure 5B). If no deviation is detected (i.e. there is little or no separation between the green and black lines shown in Figure 5B), one can assume that the subject is healthy with little risk of developing a higher rate of cognitive decline. However, if any deviation is detected (i.e. there is separation between the green and black lines shown in Figure 5B), then the next step should be carried out;
3) Step 3 - The detection of amyloid is carried out using an amyloid PET scan. If no amyloid is detected (refer to MMSE or PACC scores, etc.), then the subject is healthy with little risk of developing a higher rate of cognitive decline. However, if amyloid is detected, then they progress to the final step of diagnosis;
4) Step 4 - Depending on their soluble and aggregated T14 levels, it is possible to predict the rate of cognitive decline using the chart in Figure 9, and refer the subject to treatment if needed;
5) Step 5 - Therapeutic treatment would be advised for subjects showing propensity to develop high rates of cognitive decline in later life.

### Example 4 - AIBL cohort: Discriminatory Value of T14 levels across MMSE groups (Western blot + ELISA)

### Statistical Objective

The inventors investigated the ability of T14 levels (measured using both Western Blot and ELISA) to differentiate between and the differently classified MMSE groups (Normals (MMSE>=26) vs Cognitively Impaired (MMSE<26)).

### Data

Data from AIBL cohorts 1 & 2 (160 samples) was pooled together with subjects classified as Normal, Mild or Moderately/Severely cognitively impaired according to MMSE score (as before).

### Methods

Receiver Operating Characteristic (ROC) curves which employ multiple logistic regression to model the likelihood (log odds) of cognitive impairment across the range of T14 'cut points' were then used to assess the value of the WB and ELISA assays.

### Results

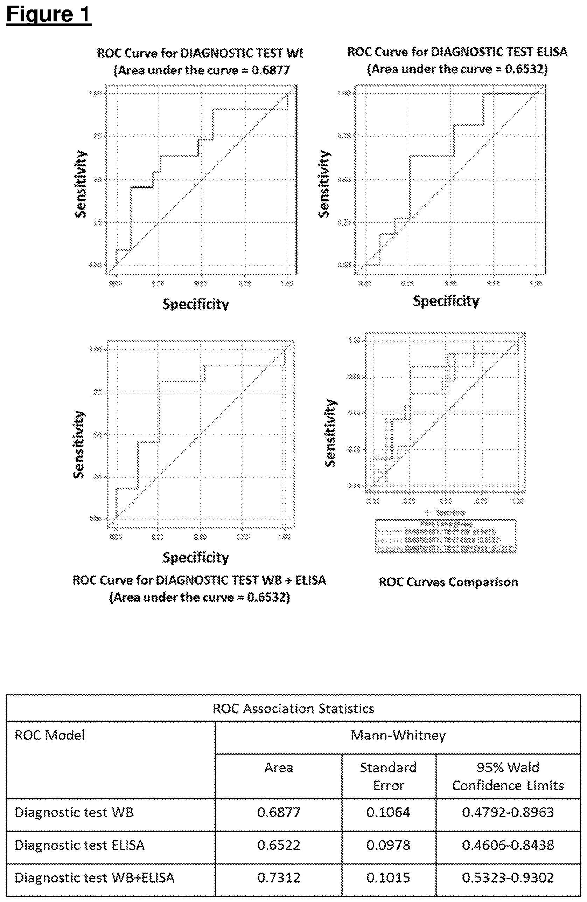

Referring to Figure 1, the ROC curves show that separately WB and ELISA methods are both reasonably sensitive and specific for detecting cognitive impairment across the two MMSE groups. However, when T14 values for both methods are used together in the model then sensitivity and specificity are improved (AUC WB+ELISA=0.732, AUC WB=0.6877, AUC ELISA=0.6522). The inventors were surprised to observe that the accuracy of the test with the combined measures was 80% sensitive and 75% specific.

### Conclusions

The two methods of ELISA and Western Blot may work in complementary fashion when assessing their ability to discriminate between normal and cognitively impaired subjects as defined by the MMSE groupings (mild/mod/severe).

Figure 8 shows the correlation between T14 levels with MMSE score in all AIBL samples, separated into MMSE score >26 for (A) aggregate T14, (B) soluble T14; MMSE score between 20-25 for C) aggregate T14, D) soluble T14; MMSE score below 20 for E) aggregate T14, F) soluble T14; recorded at the time of blood collection. Yellow highlights indicate clear statistical significance.

### Conclusions

Figure 2 shows MMSE scores in relation to disease progression, and clearly shows the non-linearity of MMSE scores with advancing impairment. Patients with severe cognitive impairments (i.e. MMSE score <20) displayed strong correlation between T14 levels (aggregated and soluble) and the degree of cognitive dysfunction. No such correlations were found for mildly impaired patients (MMSE score of 20-25) and healthy control subjects (MMSE score >26). However, the assays described herein have proved sensitive and potentially quantitative for patients with severe cognitive impairments as the P values show that the correlations between individual scores and T14 levels (measured in two ways i.e. with both WB and ELISA) are significant with only 19 patients. Since MMSE scores do not show a linear progression, it could be that the lack of significance in the healthier subjects/earlier disease stages is due to a lack of sensitivity of the MMSE in the higher range of scores.

Thus, these data corroborate the T14 assay data, thereby confirming that T14 levels (soluble and/or aggregated) can be reliably used to predict whether or not a test subject will suffer from cognitive decline in later years, way before they ever show any symptoms, as is currently the case using MMSE scoring. This means that the methods described herein which detect T14 can be used as an early warning test, such that positive steps can be taken to reduce the risk of cognitive decline (e.g. diet, life style choices), and administration of therapies, as appropriate.

### Summary

Currently there is no accepted biomarker blood test for neurodegeneration. With reference to Figures 1-9, the work described herein summarizes the analysis of 160 plasma samples assayed for the novel peptide T14 from 2 separately provided cohorts provided by the Australian Imaging, Biomarkers and Life study (AIBL) and comprising the following cases of patients: control negative amyloid, control positive amyloid, mild cognitive impairment, no memory complaints and subjective memory complaints both negative and positive for amyloid. The inventors assayed the provided plasma samples for T14 using two differently developed antibody-based methods (Western blots and ELISA). Initial exploratory investigations of the relationship between subject T14 levels and corresponding potential cognitive impairment using the Mini Mental State Examination (MMSE) test showed reasonable correlation overall, but particularly during its most sensitive phase (scores <20). The relationship (i.e. differences) between the MMSE subject groups and the levels of T14 (using Western Blots and ELISA methods) were then investigated, as were the discriminatory value of the T14 assay using Non parametric and Receiver Operating Characteristic (ROC) curve statistical methods respectively. The results clearly demonstrate that, even with such a small sample size, the T14 assays indeed show initial promise in distinguishing between the different cognitively impaired groups.

Hence, to summarise, the inventors have developed the first ever objective test for predicting whether or not a subject is prone to developing a higher rate of cognitive decline compared to a normal, healthy individual, as a result of developing a neurodegenerative disorder. Use of the quantitative T14 biomarker allows reliable detection of higher rates of cognitive decline caused by neurodegenerative disorders with a high degree of specificity and sensitivity from a non-invasive, easily repeated, cost-effective procedure, such as blood collection, and therefore allows routine screening, and early intervention with therapeutic treatment. The inventors have shown that it is possible to reliably measure levels of soluble T14 using ELISA, and levels of aggregated T14 using Western Blot. These two complementary techniques, which can be carried out independently, as in combination with each other, provide a complete picture on the way T14 levels can change in AD patients.

### Example 5 - Austria cohort: Assessing the correlation between change in T14 levels and rate of MMSE change using Western Blot

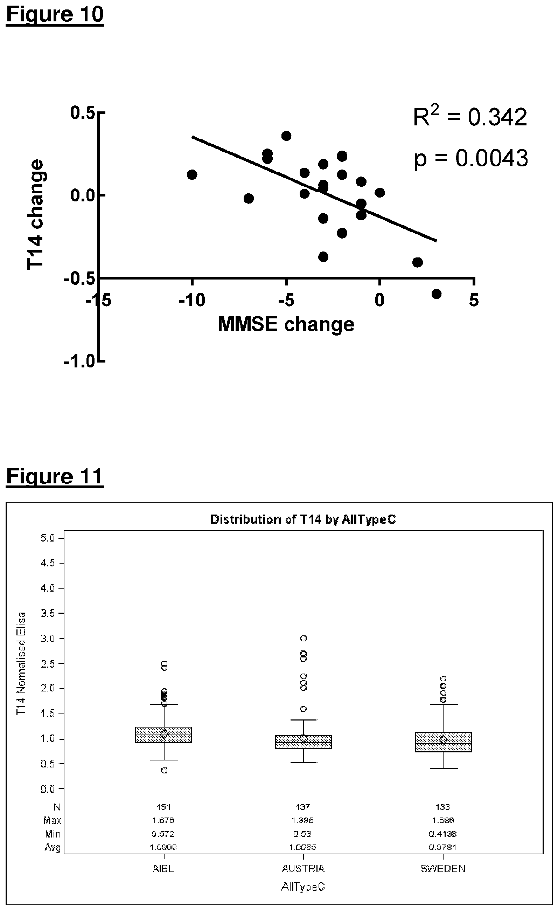

Referring to Figure 10, there is shown the correlation between change in T14 value and the rate of MMSE change in 22 AD propable patients from the Austrian cohort.

### Conclusions

- The Austrian cohort contains plasma from 119 AD probable patients and 26 AD possible patients. Each individual patient has from 1 to 6 sequential plasma samples with samples taken every 6 months after the first round of sampling (known as the baseline). MMSE tests were also taken whenever plasma was being samples.
- Sequential plasma samples from 22 out of 119 AD probable patients were tested so far for T14 using Western blotting.
- Six T14 bands were detected, same as in the AIBL cohort, and T14 all bands were quantified for each patient. Subsequently, the values were normalised against the T14 value of a healthy 40 year old external control.
- Results showed that the changes in T14 values for 22 AD probable patients are significantly correlated with their corresponding rate of MMSE changes from baselines (see Figure 10).

### Example 6 - T14 Diagnostic test ELISA normalised T14 using a pooled database with patients from AIBL, Sweden and Austria

### 1) Baseline Summaries

The T14 normalised data from the three databanks were combined and the subjects were categorised as Normal or AD. Normal subjects were categorised from the NMC, SMC, CN classified subjects in the AIBL database, AD (possibles) in the AUSTRIAN database and Controls in the SWEDEN database. All AD subjects were defined as AD or AD probables. A total of 421 subjects with baseline data were available.

Again the Age (50-59, 60-69, 70-79, >=80 years, labelled as >=50y, >=60y, >=60y, >=70y, >=80y) and MMSE (<10, 10-19, 20-25, >=26) was categorised as previously defined. NB: MMSE information was available for the Swedish data but no age information was available. Therefore, subsequently analysis that involves MMSE or clinical conditions include data from all three sources, however analysis that involves age is only carried out using the AIBL and Austrian dataset..

**Table 2 - Overall Summary By Dementia Group**

| | **T14 Normalised Elisa** | | | | | |
|---|---|---|---|---|---|---|
| | **Mean** | **Median Min Max Std N** | | | | |
| **AD** | 1.000 | 0.941 | 0.495 | 3.004 | 0.350 | 229 |
| **Normal** | 1.068 | 1.021 | 0.370 | 2.608 | 0.348 | 192 |
| **All** | 1.031 | 0.972 | 0.370 | 3.004 | 0.350 | 421 |

**Table 3 - Overall Summary By Age and MMSE Group**

| | **T14 Normalised Elisa** | | | | | |
|---|---|---|---|---|---|---|
| | **Mean** | **Median** | **Min** | **Max** | **Std** | **N** |
| **Age (yrs)** | 0.896 | 0.887 | 0.707 | 1.349 | 0.181 | 12 |
| **Age>=50y** | | | | | | |
| **Age>=60y** | 1.034 | 0.944 | 0.595 | 2.420 | 0.351 | 55 |
| **Age>=70y** | 1.032 | 0.987 | 0.370 | 2.608 | 0.313 | 155 |
| **Age>=80y** | 1.158 | 1.024 | 0.700 | 3.004 | 0.445 | 66 |
| **MMSE Categorised Baseline** | 1.064 | 0.941 | 0.598 | 3.004 | 0.447 | 54 |
| **MMSE 10-19 (Moderate)** | | | | | | |
| **Moderate MMSE 20-25 (Mild)** | 0.974 | 0.943 | 0.532 | 2.608 | 0.284 | 101 |
| **MMSE <10 (Severe)** | 1.206 | 1.420 | 0.681 | 1.518 | 0.458 | 3 |
| **MMSE >=26 (Normal)** | 1.111 | 1.048 | 0.370 | 2.679 | 0.350 | 130 |

| | | | | | | |
|---|---|---|---|---|---|---|
| NB: Small numbers in Severe MMSE group and 50-59y olds | | | | | | |

### 2) Baseline Distributions

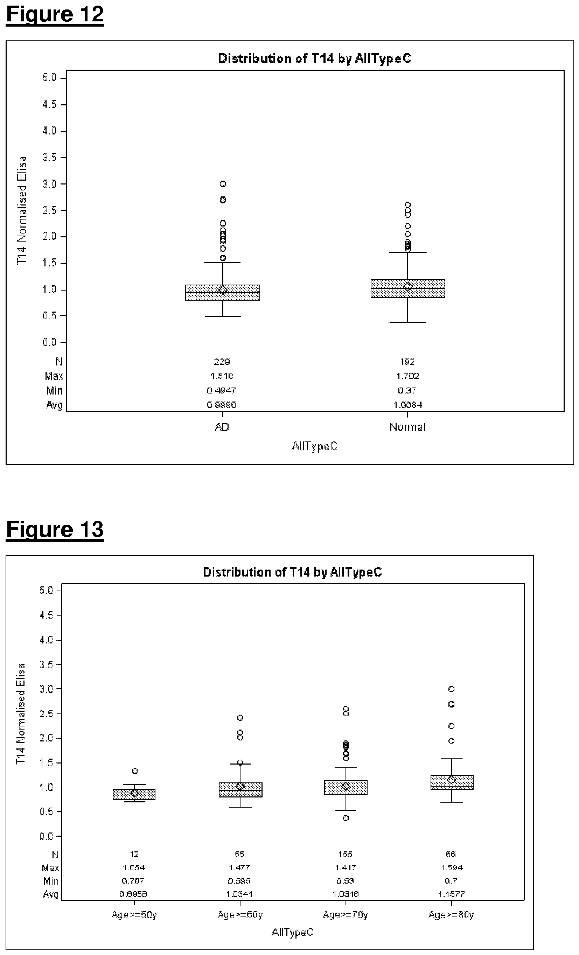

Distributions across the groups and potential covariates are explored with Box and whisker plots, as shown in Figures 11-14.

### 3) Correlation T14 By Dementia Group With Age and MMSE

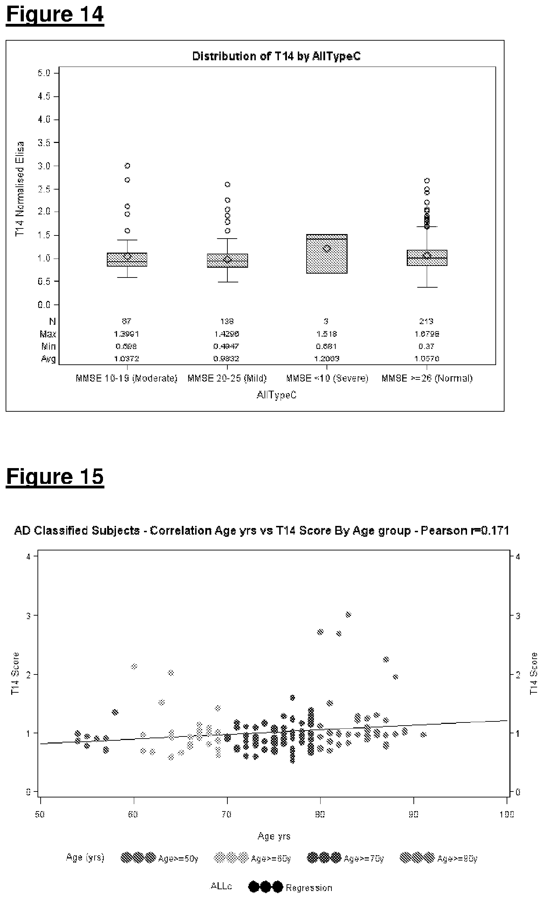

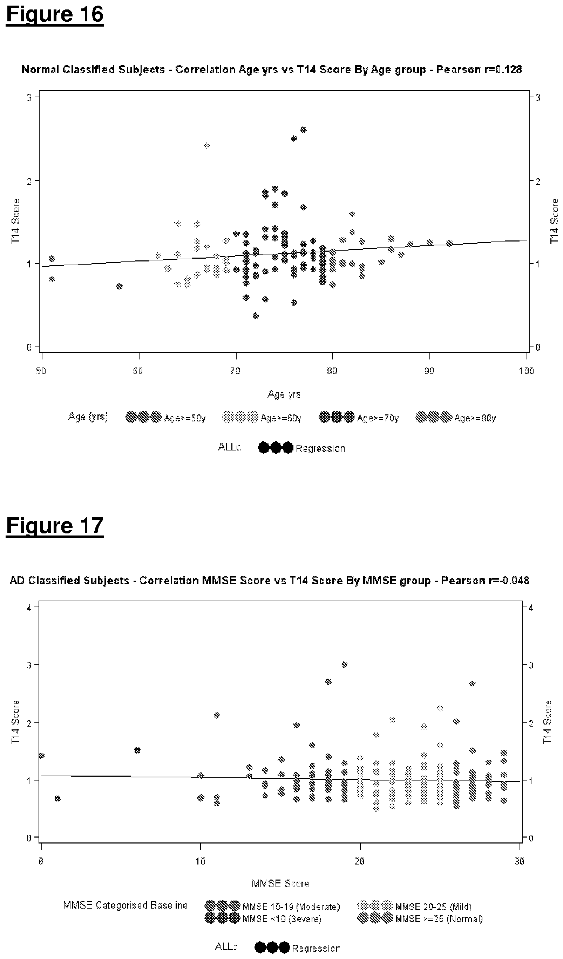

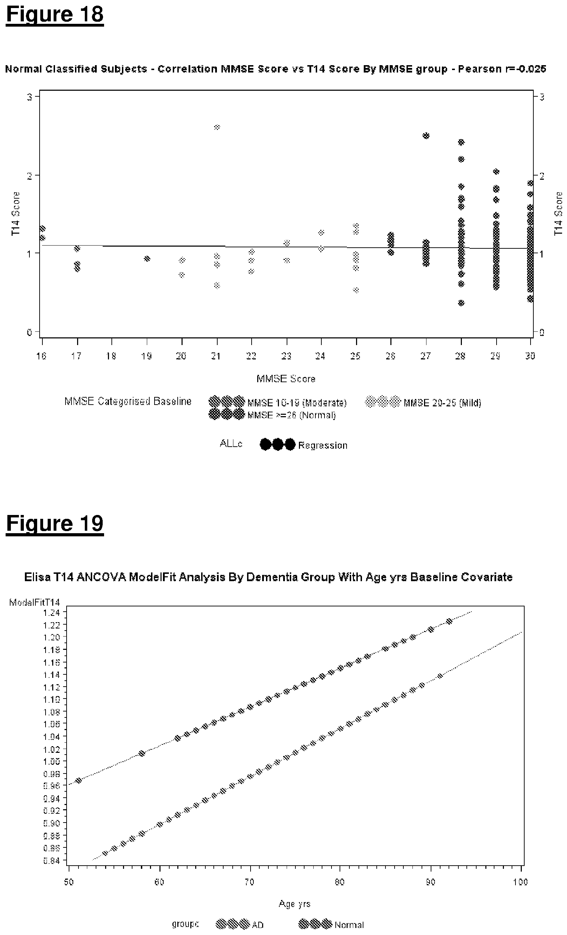

The potential relationship between T14 and Age and MMS with in each of the subjects groups has also been investigated with correlation plots. None of the Pearson correlation statistics indicated particularly strong association (r<0.2), although Age was better than MMSE, as shown in Figures 15-18.

### 4) Group Differences

Quantification of the differences in T14 between the dementia groups (AD/Normal), between the different Age groups and between the MMSE were analysed using ANOVA using main effects only.

**Table 4 - Dementia Group Differences (ANOVA) - Excluding Outliers**

| **Obs** | **TypeC** | **LSM ean** | **owerCLL** | **UpperCL** | **ProbtDiff** |
|---|---|---|---|---|---|
| **1** | AD | 0.999 | 0.954303 | 1.044963 | 0.0447 |
| **2** | Normal | 1.068 | 1.018902 | 1.117913 | - |

| | | | | | |
|---|---|---|---|---|---|
| NB: Statistically significant difference in T14 levels between AD and control groups when outliers excluded. NB: The level of error quantified in sampling repeats is 0.21 therefore observed difference may be measurement error and not necessarily real effect | | | | | |

**Table 5 - Dementia Group Differences (ANOVA) - Including Outliers**

| **Obs** | **TypeC** | **LSMean** | **LowerCL** | **UpperCL** | **ProbtDiff** |
|---|---|---|---|---|---|
| **1** | AD | 1.031 | 0.955732 | 1.107399 | 0.2247 |
| **2** | Normal | 1.101 | 1.018227 | 1.183796 | - |

| | | | | | |
|---|---|---|---|---|---|
| NB: No statistically significant differences in T14 levels between groups with outliers. | | | | | |

**Table 6 - Age Group Difference (ANOVA)**

| **Obs** | **xAGEncatc** | **LSmean** | **LowerCL** | **UpperCL** | **ProbF** |
|---|---|---|---|---|---|
| **1** | Age>=50y | 0.896 | 0.696 | 1.095 | 0.031 |
| **2** | Age>=60y | 1.034 | 0.941 | 1.127 | · |
| **3** | Age>=70y | 1.032 | 0.976 | 1.087 | · |
| **4** | Age>=80y | 1.158 | 1.073 | 1.243 | · |

| | | | | | |
|---|---|---|---|---|---|
| NB: Statistically significant difference in T14 levels between age groups. Appears to be a linear trend with T14 increasing with age irrespective of disease group. (Level of error quantified in repeat sampling was 0.21 therefore may be real effect and not measurement error) | | | | | |

**Table 7 - MMSE Group Difference (ANOVA)**

| **Obs** | **xMMSEcatc** | **LSmean** | **LowerCL** | **UpperCL** | **ProbF** |
|---|---|---|---|---|---|
| **1** | MMSE >=26 (Normal) | 1.058 | 1.010 | 1.105 | 0.2067 |
| **2** | MMSE 20-25 (Mild) | 0.983 | 0.925 | 1.042 | · |
| **3** | MMSE 10-19 (Moderate) | 1.037 | 0.953 | 1.121 | · |
| **4** | MMSE <10 (Severe) | 1.206 | 0.810 | 1.603 | · |

| | | | | | |
|---|---|---|---|---|---|
| NB: No statistically significant differences in T14 levels between MMSE groups | | | | | |

### 5) T14 Analysis Of Group Differences With Age Baseline Covariate

The influence of covariates such as Age and MMSE was also assessed using ANCOVA to assess the relationship between T14 and possible prognostic factors. Displays of model estimates vs baseline covariates for the different groups are useful indicators (assuming the model is reasonable). Model predicted T14 estimates are presented against the baseline value according to the different groups.

**Table 8 - ANCOVA Model With Dementia Group And Age Covariate: Statistics**

| **Source** | **DF** | **Type III SS** | **Mean Square** | **F Value** | **Pr > F** |
|---|---|---|---|---|---|
| **Group** | 1 | 0.78930078 | 0.78930078 | 6.51 | 0.0112 |
| **Age** | 1 | 0.84134171 | 0.84134171 | 6.94 | 0.0089 |

| | | | | | |
|---|---|---|---|---|---|
| NB: Group again statistically different plus Age statistically significant covariate in explaining T14 differences (as continuous or fixed effect). There is no significant Group*Covariate interaction so consistent effect as shown in Figure 19. | | | | | |

**Table 9 - ANCOVA Model With Dementia Group And MMSE Covariate: Statistics**

| **Source** | **DF** | **Type III SS** | **Mean Square** | **F Value** | **Pr > F** |
|---|---|---|---|---|---|
| **Group** | 1 | 0.52959011 | 0.52959011 | 4.34 | 0.0377 |
| **MMSE** | 1 | 0.07730034 | 0.07730034 | 0.63 | 0.4263 |

| | | | | | |
|---|---|---|---|---|---|
| NB: Group again statistically different in explaining T14 differences but not MMSE covariate (either as continuous or fixed effect). There is no significant Group*Covariate interaction so consistent effect as shown in Figure 20. | | | | | |

### 6) Overall Model Selection

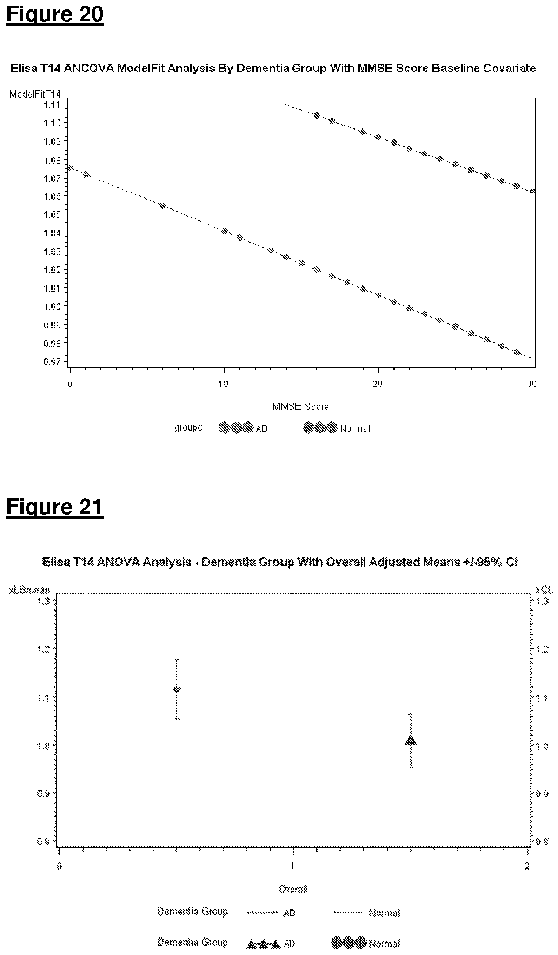

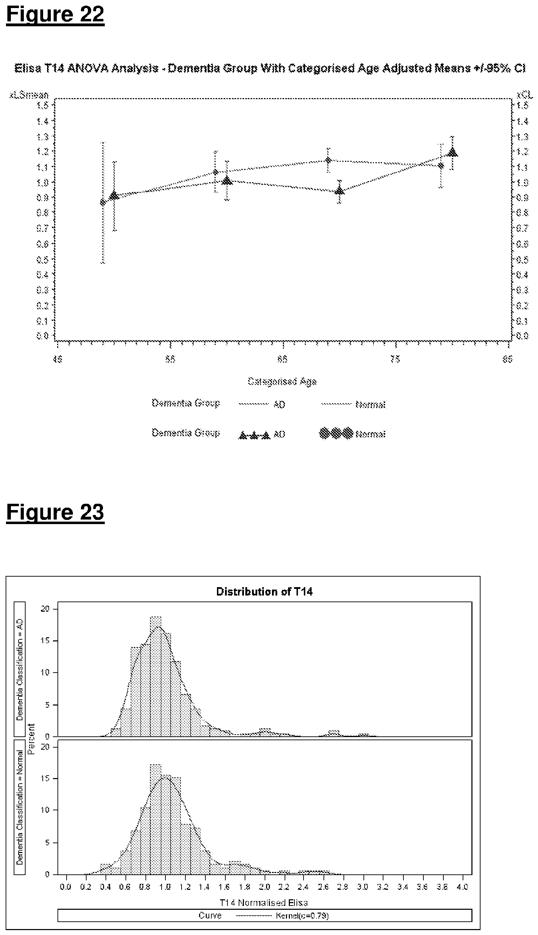

From the various models evaluated a final ANCOVA model including Group (AD/Normal) as a fixed effect and Age as an explanatory continuous covariate (without interaction) was selected. Both Group and Age were statistically significant contributors to the model but the interaction when considered continuous was not. Adjusted mean (+/- 95%CIs) model estimates are presented graphically as in Figures 21 and 22, both for the Groups overall and for the Groups effects according to categorised Age.

NB: Not described here but other demographic covariates (Duration, Education, Sex etc) were assessed but none proved informative.

**Table 10 - Overall Group Differences: Adjusted Means (+/-95% CI)**

| **Obs** | **Group** | **LSmean** | **LowerCL** | **UpperCL** | **ProbF** |
|---|---|---|---|---|---|
| **1** | Normal | 1.115 | 1.054 | 1.176 | .0089 |
| **2** | AD | 1.009 | 0.955 | 1.063 | |

| | | | | | |
|---|---|---|---|---|---|
| NB: Adjusted mean difference (95%CI) between AD-Control = -0.10 (-1.8 to -0.02). (The ratio of AD/Control expressed as a percentage was 90.0% (84.4% to 96.0%) representing a difference between the two groups of 10%). Consistent differences were noted with log transformed T14 using log ANCOVA and also using CMH test. Significant differences were notable between AD-Control for the 70-79 age group. | | | | | |

**Table 11 - Group Differences According To Age : Adjusted Means (+/-95% CI)**

| | **Obs Group** | **AGE** | **LSmean** | **LowerCL** | **UpperCL** | **ProbF** |
|---|---|---|---|---|---|---|
| **1** | Normal | Age>=50y | 0.864 | 0.472 | 1.255 | 0.0423 |
| **2** | | Age>=60y | 1.064 | 0.931 | 1.197 | . |
| **3** | | Age>=70y | 1.140 | 1.060 | 1.219 | . |
| **4** | | Age>=80y | 1.106 | 0.968 | 1.245 | |
| **5** | AD | Age>=50y | 0.906 | 0.680 | 1.132 | . |
| **6** | | Age>=60y | 1.007 | 0.881 | 1.133 | |
| **7** | | Age>=70y | 0.936 | 0.861 | 1.011 | . |
| **8** | | Age>=80y | 1.187 | 1.082 | 1.292 | . |

| | | | | | | |
|---|---|---|---|---|---|---|
| NB: Statistical significance only refers to Group^{∗}Age interaction when Age considered categorical only (less useful in model as less information used when categorical) | | | | | | |

### 7) Diagnostic Accuracy

The diagnostic accuracy of T14 in correctly classifying subjects as AD or Normal is assessed using ROC curves (logistic regression). Initial analyses included classification of the subject groups according to T14 level alone and then with age added as a covariate. Subgroup analyses then involved ROC analyses for the four age groups separately. Histograms of the T14 distributions by group are also given to assist with explanation of the likely discriminatory ability of the model. In all cases AUCs indicating the strength of sensitivity and specificity were modest.

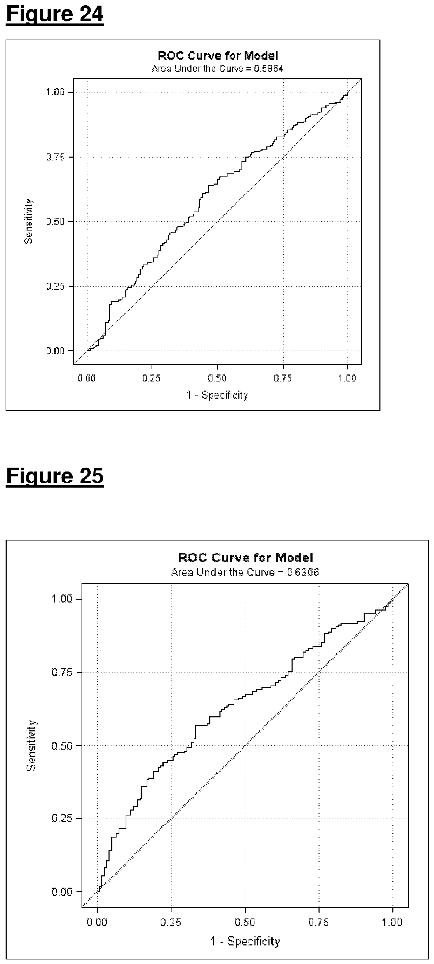

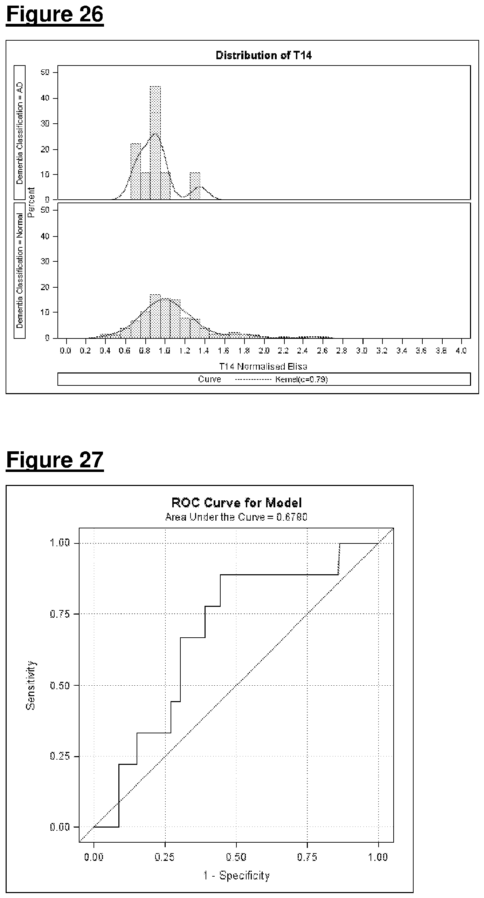

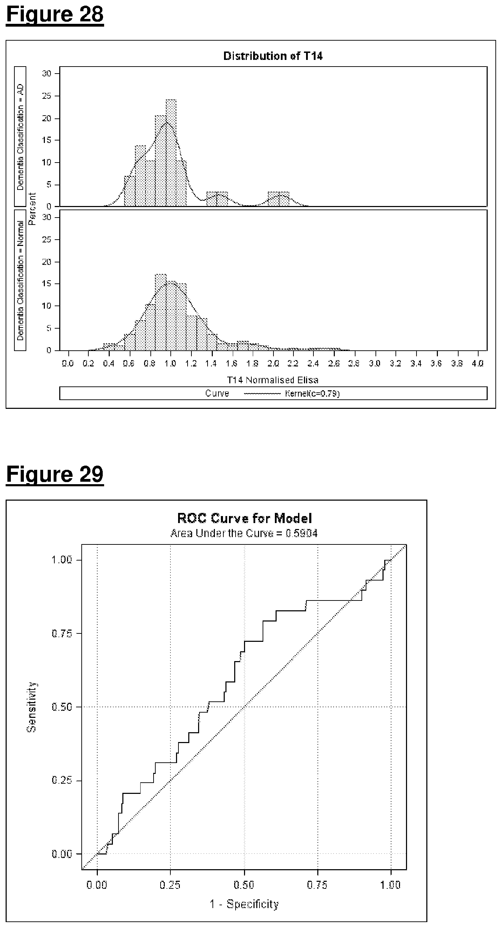

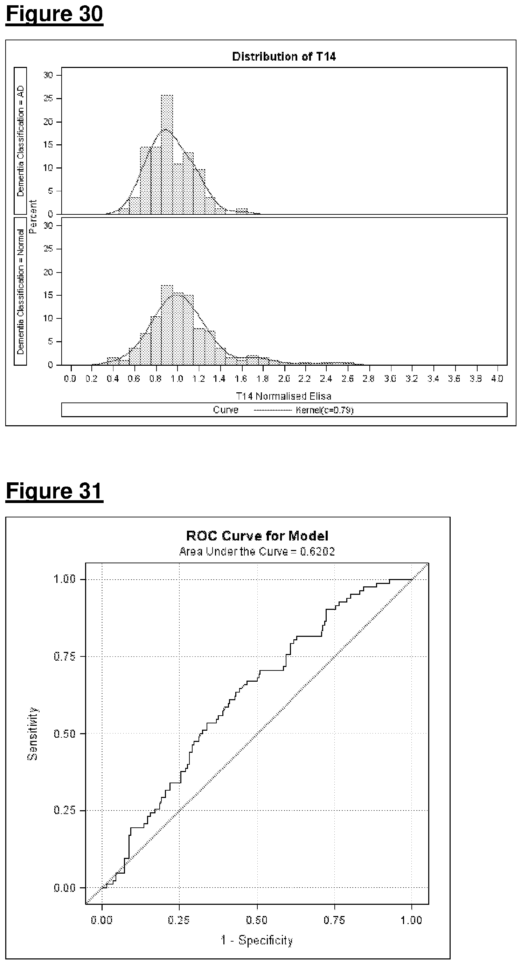

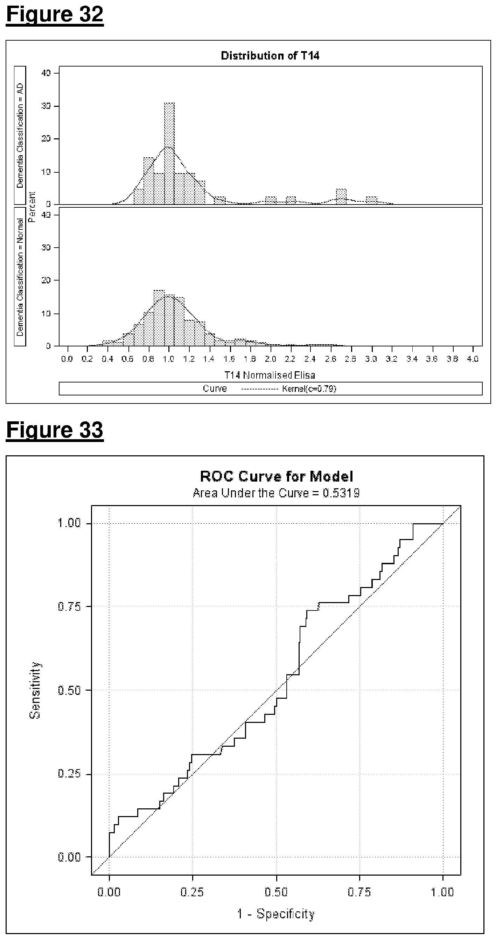

Referring to Figures 23-33, there are shown Distribution curves for T14 across different subject groups and subgroups with matching ROC (Receive Operator Curves). The ROC analysis model makes it possible to specify different "cut points" to dichotomise the classification of subjects to the different defined groups and therefore assess the accuracy of T14 in correctly matching subjects to the clinically defined groupings.

### Example 7 - Detection of T14 and T14 fragments for diagnosis and prognosis

The inventors set out to determine if fragments of T14 (AEFHRWSSYMVHWK, i.e. SEQ ID No:3) are also detectable, and can act as diagnostic or prognostic markers used in accordance with the disclosure.

### Materials and Methods

Fragments that were tested were:
(i) SYMVHWK (SEQ ID No:7, i.e. T7);
(ii) SSYMVHWK (SEQ ID No: 8, i.e. T8);
(iii)WSSYMVHWK (SEQ ID No: 9, i.e. T9);
(iv) RWSSYMVHWK (SEQ ID No: 10, i.e. T10);
(v) HRWSSYMVHWK (SEQ ID No: 11, i.e. T11);
(vi)FHRWSSYMVHWK (SEQ ID No: 12, i.e. T12); and
(vii) EFHRWSSYMVHWK (SEQ ID No: 13, i.e. T13).

Each of the above fragments are derived from T14 but lacked a certain number of N-terminal amino acids. T13, for example, is identical to T14 except it lacks the N-terminal 'A' residue, T12 lacks the N-terminal 'AE' residues, and so on. T7 lacks the seven N-terminal residues, T6 lacks the eight N-terminal residues, and T5 lacks the nine N-terminal residues.

### ELISA for the T14 peptide variants (T5-T14)

The standard curves were run in triplicate and the peptide variants were run in quadruplicate. The peptide variants were diluted to a concentration of 150nM in PBS Buffer. The standard curve ranged from 12 to 150 nM of T14, and the blank was PBS buffer alone. Briefly, 96-well immunoplates (NUNC) were coated with 100 *µ*l/well of peptide variant or standard T14, covered with parafilm and incubated overnight at 4°C. The following day the sample was removed by flicking the plate over a sink with running water, and 200 *µ*l of the blocking solution containing 2% bovine serum albumin (BSA) in Tris-buffered saline and Tween 20 (TBS-T) was added and incubated for 4h at room temperature. Blocking solution was then removed and 100 *µ*l of antibody (described in WO 2016/156803), diluted in blocking solution to 1µg/ml, was added and incubated overnight at 4 °C. The primary antibody was removed the next day and wells were washed 3 times with 200 *µ*l of TBS-T. After 100 µl of secondary enzyme-conjugated antibody (see WO 2016/156803) diluted in blocking solution to 0.1 µg/ml was added and incubated for 2 h at room temperature; the plate was covered with parafilm during all incubations. After 2 h, the plate was washed 4 times with TBS-T. The addition of 3,3,5,5-tetramethylbenzidine started the colour reaction. The reaction was stopped 20 min later with stopping solution containing 2 M H2SO4, and the absorbance was measured at 450 nm in a Versamax plate reader (Molecular Devices, Wokingham, UK).

### Results

The inventors focused much of this work by detecting T14 (SEQ ID No:3), and using T14 is a prognostic biomarker for predicting the rate at which a subject will suffer from cognitive decline, or for detecting a pre-disposition thereto. However, as shown in Figure 34, it is clear that shorter peptides based on T14 are also readily detectable using the antibody described herein, i.e. peptides T7-T14 are detectable, provided they include the C-terminal epitope. These shorter peptides are missing the corresponding N-terminal amino acids.

## Claims

1. An *in vitro* method for predicting the rate at which a subject will suffer from cognitive decline caused by Alzheimer's disease, the method comprising:-
(a) analysing, in a plasma sample obtained from a test subject, the concentration of a soluble peptide T14 consisting of SEQ ID No:3; and
(b) comparing this concentration with a reference value from a control population for concentrations of a soluble peptide consisting of SEQ ID No:3,
wherein a lower concentration of soluble peptide consisting of SEQ ID No:3, from the test subject, relative to the respective reference value, suggests that the subject will suffer from a higher rate of cognitive decline, wherein the concentration is 110µM or lower.

2. A method according to claim 1, wherein the age of the subject is at least 30, 40, 50, 60 or 70 years old.

3. A method according to any preceding claim, wherein an immunoassay, optionally ELISA, is used to detect soluble T14 peptide in the sample.

4. A method according to any preceding claim, wherein the reference value is calculated from a plurality of individuals who are between 60 and 90 years old, some of whom are healthy and some of whom are showing at least some cognitive decline, and/or wherein calculation of the reference value comprises a step of designating members of the reference population as either Amyloid positive or Amyloid negative, optionally wherein such designation is achieved by assessing beta-amyloid relative values on the BeCKeT scale, wherein an Amyloid negative patient has a BeCKeT value of < 1.4, and an Amyloid positive subject has a BeCKeT value of >1.4.

5. A method according to any preceding claim, wherein the method comprises measuring the rate of cognitive decline by a Mini Mental State Examination (MMSE) score, optionally wherein the method comprises measuring the rate of cognitive decline by a Preclinical Alzheimer Cognitive Composite (PACC) score.

6. A method according to any preceding claim, wherein:
i) the method comprises a step of age-adjusting the soluble T14 concentrations from the test subject against the corresponding reference value; and/or
ii) the method comprises a step of detecting the presence or absence of beta-amyloid in the sample, optionally using PET imaging, and then designating the test subject as either Amyloid positive or Amyloid negative; and/or
iii) the method comprises the use of a combination of age-adjusted T14 levels and amyloid beta imaging to predict the likelihood of a higher rate of cognitive decline.

7. *In vitro* use of a peptide consisting of SEQ ID No:3, as a prognostic biomarker for predicting the rate of cognitive decline caused by Alzheimer's disease.

## Patentansprüche

1. *In-vitro*-Verfahren zur Vorhersage der Rate, mit der ein Subjekt an kognitivem Verfall verursacht durch Alzheimer-Krankheit leiden wird, wobei das Verfahren Folgendes umfasst:
(a) Analysieren, in einer Plasmaprobe, die von einem Testsubjekt erhalten wird, der Konzentration eines löslichen Peptids T14, das aus SEQ ID No. 3 besteht; und
(b) Vergleichen dieser Konzentration mit einem Referenzwert aus einer Kontrollpopulation für Konzentrationen eines löslichen Peptids, das aus SEQ ID No. 3 besteht,
wobei eine niedrigere Konzentration an löslichem Peptid, das aus SEQ ID No. 3 besteht, von dem Testsubjekt relativ zu dem jeweiligen Referenzwert darauf schließen lässt, dass das Subjekt an einer höheren Rate an kognitivem Verfall leiden wird, wobei die Konzentration 110 µM oder niedriger ist.

2. Verfahren nach Anspruch 1, wobei das Alter des Subjekts zumindest 30, 40, 50, 60 oder 70 Jahre ist.

3. Verfahren nach einem vorhergehenden Anspruch, wobei ein Immunoassay, optional ELISA, verwendet wird, um lösliches T14-Peptid in der Probe nachzuweisen.

4. Verfahren nach einem vorhergehenden Anspruch, wobei der Referenzwert aus einer Vielzahl von Personen berechnet wird, die zwischen 60 und 90 Jahre alt sind, von denen einige gesund sind und von denen einige zumindest einen gewissen kognitiven Verfall zeigen, und/oder wobei Berechnung des Referenzwertes einen Schritt des Bezeichnens von Mitgliedern der Referenzpopulation als entweder Amyloid-positiv oder Amyloid-negativ umfasst, wobei optional eine solche Bezeichnung durch Bewerten von relativen Beta-Amyloid-Werten auf der BeCKeT-Skala erreicht wird, wobei ein Amyloid-negativer Patient einen BeCKeT-Wert von <1,4 aufweist und ein Amyloid-positives Subjekt einen BeCKeT-Wert von >1,4 aufweist.

5. Verfahren nach einem vorhergehenden Anspruch, wobei das Verfahren Messen der Rate an kognitivem Verfall durch einen Mini-Mental-State-Examination-(MMSE)Score umfasst, wobei optional das Verfahren Messen der Rate an kognitivem Verfall durch einen Preclinical-Alzheimer-Cognitive-Composite-(PACC)Score umfasst.

6. Verfahren nach einem vorhergehenden Anspruch, wobei:
i) das Verfahren einen Schritt der Altersanpassung der löslichen T14-Konzentrationen von dem Testsubjekt gegenüber dem entsprechenden Referenzwert umfasst; und/oder
ii) das Verfahren einen Schritt des Nachweisens des Vorhandenseins oder Nichtvorhandenseins von Beta-Amyloid in der Probe, optional unter Verwendung von PET-Bildgebung, und dann Benennens des Testsubjekts als entweder Amyloid-positiv oder Amyloid-negativ umfasst; und/oder
iii) das Verfahren die Verwendung einer Kombination aus altersangepassten T14-Spiegeln und Amyloid-Beta-Bildgebung umfasst, um die Wahrscheinlichkeit einer höheren Rate an kognitivem Verfall vorherzusagen.

7. *In-vitro*-Verwendung eines Peptids, das aus SEQ ID No. 3 besteht, als prognostischer Biomarker zur Vorhersage der Rate an kognitivem Verfall, der durch Alzheimer-Krankheit verursacht wird.

## Revendications

1. Méthode *in vitro* permettant de prédire le taux auquel un sujet souffrira d'un déclin cognitif provoqué par la maladie d'Alzheimer, la méthode comprenant :-
(a) l'analyse, dans un échantillon de plasma obtenu à partir d'un sujet testé, de la concentration d'un peptide T14 soluble constitué par SEQ ID n° : 3 ; et
(b) la comparaison de cette concentration à une valeur de référence provenant d'une population témoin pour les concentrations d'un peptide soluble constitué par SEQ ID n° : 3,
une concentration inférieure de peptide soluble constitué par SEQ ID n° : 3, du sujet testé, par rapport à la valeur de référence respective, suggérant que le sujet souffrira d'un taux plus élevé de déclin cognitif, la concentration étant de 110 µM ou moins.

2. Méthode selon la revendication 1, l'âge du sujet étant d'au moins 30, 40, 50, 60 ou 70 ans.

3. Méthode selon l'une quelconque des revendications précédentes, un dosage immunologique, éventuellement ELISA, étant utilisé pour détecter le peptide T14 soluble dans l'échantillon.

4. Méthode selon l'une quelconque des revendications précédentes, ladite valeur de référence étant calculée à partir d'une pluralité d'individus qui ont entre 60 et 90 ans, dont certains sont en bonne santé et dont certains présentent au moins un certain déclin cognitif, et/ou le calcul de la valeur de référence comprenant une étape de désignation des membres de la population de référence comme étant soit positifs aux amyloïdes ou négatifs aux amyloïdes, éventuellement une telle désignation étant obtenue en évaluant les valeurs relatives de bêta-amyloïde sur l'échelle BeCKeT, un patient négatif aux amyloïdes comportant une valeur BeCKeT de < 1,4, et un sujet positif aux amyloïdes comportant une valeur BeCKeT > 1,4.

5. Méthode selon l'une quelconque des revendications précédentes, ladite méthode comprenant la mesure du taux de déclin cognitif par un score MMSE (Mini Mental State Examination), éventuellement ladite méthode comprenant la mesure du taux de déclin cognitif par un score PACC (Preclinical Alzheimer Cognitive Composite).

6. Méthode selon l'une quelconque des revendications précédentes :
i) ladite méthode comprenant une étape d'ajustement en fonction de l'âge des concentrations de T14 soluble du sujet testé par rapport à la valeur de référence correspondante ; et/ou
ii) ladite méthode comprenant une étape de détection de la présence ou de l'absence de bêta-amyloïde dans l'échantillon, éventuellement à l'aide d'une imagerie TEP, puis de désignation du sujet testé comme étant positif aux amyloïdes ou négatif aux amyloïdes ; et/ou
iii) ladite méthode comprenant l'utilisation d'une combinaison de taux de T14 ajustés en fonction de l'âge et d'imagerie de la bêta-amyloïde pour prédire la probabilité d'un taux plus élevé de déclin cognitif.

7. Utilisation *in vitro* d'un peptide constitué par SEQ ID n° : 3, en tant que biomarqueur de pronostic pour prédire le taux de déclin cognitif provoqué par la maladie d'Alzheimer.
